(19) Europäisches Patentamt
European Patent Office
Office européen des brevets

(11) **EP 3 139 967 B1**

(12) **EUROPEAN PATENT SPECIFICATION**

(45) Date of publication and mention
of the grant of the patent:
**08.04.2020 Bulletin 2020/15**

(51) Int Cl.:
*A61K 51/12* *(2006.01)*      *A61P 35/00* *(2006.01)*
*A61N 5/10* *(2006.01)*      *A61K 9/00* *(2006.01)*
*A61B 18/00* *(2006.01)*

(21) Application number: **15788648.2**

(22) Date of filing: **07.05.2015**

(86) International application number:
**PCT/AU2015/000268**

(87) International publication number:
**WO 2015/168726 (12.11.2015 Gazette 2015/45)**

(54) **MICROPARTICLES FOR TREATING RENAL CELL CARCINOMA**

MIKROPARTIKEL FÜR BEHANDLUNG VON NIERENZELLENKARZINOM

MICROPARTICULES POUR TRAITEMENT DU CARCINOME DES CELLULES RÉNALES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.05.2014 AU 2014901697**

(43) Date of publication of application:
**15.03.2017 Bulletin 2017/11**

(73) Proprietor: **Sirtex Medical Pty Ltd
St Leonards, NSW 2065 (AU)**

(72) Inventor: **CADE, David
North Sydney, New South Wales 2060 (AU)**

(74) Representative: **Schiweck Weinzierl Koch
Patentanwälte Partnerschaft mbB
Ganghoferstraße 68 B
80339 München (DE)**

(56) References cited:
**WO-A1-2006/002488      US-A1- 2004 258 614
US-A1- 2012 070 371**

• HAMOUI ET AL: "Radioembolization ot renal cell carcinoma using yttrium-90 microspheres", JOURNAL OF VASCULAR AND INTERVENTIONAL RADIOLOGY, ELSEVIER, AMSTERDAM, NL, vol. 24, no. 2, 1 February 2013 (2013-02-01), pages 298-300, XP008185064, ISSN: 1051-0443

• Anonymous: "Pilot study of selective internal radiation therapy (SIRT) with yttrium-90 resin microspheres (SIR-Spheres microspheres) in patients with renal cell carcinoma ( STX0110), Trial ID ACTRN12610000690055", .anzctr., 23 July 2013 (2013-07-23), pages 1-4, XP055360270, Retrieved from the Internet: URL:https://www.anzctr.org.au/Trial/Regist ration/TrialReview.aspx?id=335845 [retrieved on 2017-03-30]

• C J DUCKETT ET AL: "PROCEEDINGS OF THE TUMOR BOARD OF THE CHILDREN'S HOSPITAL OF PHILADELPHIA Brachytherapy for Residual Intra-Renal Wilms' Tumor", MEDICAL AND PEDIATRIC ONCOLOGY, vol. 28, 1 January 1997 (1997-01-01), pages 316-320, XP055360279,

• HAMOUI, N. ET AL.: 'Radioembolization ot renal cell carcinoma using yttrium-90 microspheres' J. VASC. INTERV. RADIOL. vol. 3, 2013, pages 298 - 300, XP008185064

• LANG, E. ET AL.: 'Management of primary and metastatic renal cell carcinoma by transcatheter embolization with iodine 125' CANCER. vol. 62, no. 2, 1988, pages 274 - 82, XP055235615

• 'Pilot study of selective internal radiation therapy (SIRT) with yttrium-90 resin microspheres (SIR-Spheres microspheres) in patients with renal cell carcinoma (STX0110), Trial ID ACTRN12610000690055' 23 July 2015, XP055360270 Retrieved from the Internet: <URL:https://www.anzctr.org.au/Trial/Regist ration/TrialReview.aspx?id=335845> [retrieved on 2013-07-19]

- 'Package Insert, TheraSphere® Yttrium-90 Glass Microspheres (USA)', [Online] 23 July 2015, XP055235616 Retrieved from the Internet: <URL:http://www.therasphere.com/physicians-package- insert/TS_PackageInsert_ USA _v 12.pdf>
- MACKIE, S. ET AL.: 'Superselective radioembolization of the porcine kidney with Yttrium-90 resin microspheres: A feasibility, safety and dose-ranging study.' CARDIOVASCULAR AND INTERVENTIONAL RADIOLOGY vol. 33, no. 2, 2010, page 265, XP027553852
- DUCKETT, C. ET AL.: 'Brachytherapy for Residual Intra-Renal Wilms' Tumor' MEDICAL AND PEDIATRIC ONCOLOGY vol. 28, 1997, pages 316 - 320, XP055360279
- KWIATKOWSKI, J.: 'Perioperative brachytherapy as an additional therapeutic option in patients with renal cell carcinoma (RCC), either inoperable or after completed percutaneous radiotherapy' ANTICANCER RESEARCH vol. 19, no. 2C, 1999, pages 1597 - 1599, XP008184858

**Description**

**TECHNICAL FIELD**

This invention relates to microparticles for treating Renal Cell Carcinomas (**RCC**) using Selective Internal Radiation Therapy (**SIRT**). In particular, it relates to a method for determining the dose of radioactive microparticles, which may be of any form, for treating RCC's.

**BACKGROUND ART**

RCC accounts for around 3% of all cancers in the United States, with approximately 58,000 new cases and 13,000 deaths recorded in 2009. Over the past 20 years, there has been a five-fold increase in the incidence of RCC and a two-fold increase in mortality. Widespread use of diagnostic ultrasound and CT imaging has resulted in approximately 60% of cases of RCC being diagnosed incidentally. Most diagnoses (two-thirds) are made in men (median age 65 years).

Laparoscopic, rather than open, radical nephrectomy is the mainstay of treatment for patients with localised disease and with a normal contralateral kidney. However, with around 20% of small renal masses (<4cm) being benign, nephron-sparing surgery is now recommended for small renal masses due to the lower risk of chronic kidney disease and equivalent long-term survival in selected cases compared with radical nephrectomy.

Further indications for nephron-sparing surgery include patients with a solitary kidney, bilateral tumours, significant risk factors for chronic kidney disease and hereditary renal cancer syndromes. Still, open partial nephrectomy remains the standard of care for more complex cases including patients with centrally located tumours, or solitary kidneys. For larger tumours, partial nephrectomy may be associated with a higher risk of local recurrence, however long-term data on outcomes are currently relatively lacking.

Surgery is not appropriate in all cases and active surveillance may be employed for small renal masses in elderly patients or in those with significant comorbidity. Compared with younger patients, these lesions have a lower risk of early progression with an annual growth rate of around 0.3cm. Approximately 46% of tumours <1cm and 20% of tumours measuring 3cm - 3.9cm ultimately prove to be benign on histological examination. Moreover, malignant small renal masses tend to be of a lower grade than larger symptomatic lesions. Overall progression in selected patients is around 34% with a 2% risk of developing metastases.

Metastatic disease is found at diagnosis or develops after definitive treatment in 30% - 40% of patients with RCC. Most of these tumours are large, locally advanced and attached to the renal vein or regional lymph nodes. Current treatment strategies involve cytoreductive nephrectomy in order to reduce the tumour burden, reduce tumour complications during systemic therapy, provide definitive histology, and for palliation. However, mortality ranges from 2% to 11% and morbidity is high with around 38% of patients unfit for systemic therapy postoperatively. Moreover, the benefits are modest with an approximate 3-month median survival advantage for patients undergoing cytoreductive surgery and subsequent systemic therapy versus systemic therapy alone.

Ablative techniques, primarily radiofrequency ablation (RFA) and cryotherapy offer an alternative option to surgical resection for small renal masses in patients who have progressed whilst under active surveillance or who are unfit for surgery. Studies of RFA and cryotherapy show reduced morbidity and increased quality-of-life (QoL) compared to nephrectomy. Both options can be undertaken laparoscopically or percutaneously depending on tumour size, location and proximity to adjacent organs. Five-year survival rates comparable to nephrectomy have been reported in selected cases but there is a lack of long-term data, an increased risk of local recurrence and lack of tissue for tumour staging.

As advanced RCC carries a poor prognosis, many adjuvant treatment options have been investigated in an attempt to improve patient outcomes. Standard chemotherapy regimens have failed consistently to produce any benefit. However, several new-targeted therapies have been developed as a result of an improved understanding of the molecular and genetic pathways involved in renal carcinogenesis. The multi-targeted tyrosine kinase inhibitors (**TKIs**), sunitinib and sorafenib and the mammalian target of rapamycin (**mTOR**) inhibitor, temsirolimus have all shown benefits in progression-free survival and response rates compared with traditional immunotherapy. These new drugs represent an important development in the treatment of advanced RCC, but the benefits remain modest.

Neoadjuvant and adjuvant radiotherapy for renal cancer were investigated in the 1960's and 70's. Some initial studies suggested improved outcomes but subsequent investigation showed no survival advantage over surgery alone. Moreover, traditional external beam radiotherapy (**EBRT**) appeared to have little effect on local recurrence or development of metastatic disease and adverse effects on adjacent organs including liver and bowel were problematic. Consequently, adjuvant radiotherapy was largely abandoned in conventional medical practice because local recurrence was rare postoperatively. However, more recent advances in tumour-directed radiotherapy approaches including stereotactic body radiotherapy have recently yielded promising results as a new nephron-sparing, non-invasive approach for the treatment of advanced RCC and in patients with only one functioning kidney.

Despite the many refinements in surgical techniques and new targeted pharmacologic agents, renal tumours remain one of the most lethal urological cancers. Adjuvant treatment options are limited and there is a clear need for further research and new treatment approaches in this field.

Selective internal radiation therapy (SIRT), which is the intra-arterial delivery of radioactive microparticles to tumours, has an established therapeutic role in the management of inoperable primary and metastatic liver tumours. However, the utility of SIRT for the management of RCC remains largely unexplored and unknown in light of whether the same endovascular principles may be deployed and more relevantly what dose is required in cases of renal trauma and in the management of a range of benign and malignant conditions. For example, document "Superselective radio-embolization of the porcine kidney with Yttrium-90 resin microspheres: A feasibility, safety and dose-ranging study."(Mackie S. et. al.CARDIOVASCULAR AND INTERVENTIONAL RADIOLOGY, vol. 33, no. 2, 2010, pp 285-290) discusses a radio embolisation procedure for treatment of RCC, wherein $^{90}$Y microparticles are applied.

It is against this background that the present invention has been developed.

**SUMMARY OF INVENTION**

The inventors have revealed that the method of the invention can provide an effective treatment in cases of renal trauma and in the management of a range of benign and malignant renal conditions.

According an aspect of the disclosure there is provided a method of treatment for renal neoplastic conditions in a subject comprising subjecting the subject to SIRT, wherein (i) the prescribed activity of the irradiated microparticles used in the selective internal radiation therapy is 0.02 to 3.5 GBq and (ii) the therapy delivers between 75 and 800 Gy to the site of treatment in the kidney. Preferably the site of treatment is restricted the neoplasia.

In a second aspect of the disclosure there is provided a method for treating a kidney neoplasia in a subject in need of treatment comprising the step of: administering to the kidney neoplasia an amount of microparticles that delivers a radiation dose between 100 and 600Gy. The microparticles used in the method should are preferably suitable for selective internal radiation therapy. Those particles will ideally present a level of radioactivity that is between about 0.02 to 3.5 GBq. More preferably the radiation level presented is capped at a maximum of 3 GBq. In an alternate form the radioactivity of the microparticles used in the SIRT is calculated by determining tumour volume and adjusting the amount of the radioactive microparticles, having regard to tumour volume, to deliver to the kidney neoplasia a radiation dose between 100 and 600Gy

Other aspects and advantages of the invention will become apparent to those skilled in the art from a review of the ensuing description of several non-limiting embodiments thereof.

The invention is defined in the claims, other embodiments as well as therapeutic and surgical methods are provided for illustrative purposes only.

**DETAILED DESCRIPTION OF THE INVENTION**

Throughout this specification, unless the context requires otherwise, the word "comprise", or variations such as "comprises" or "comprising", will be understood to imply the inclusion of a stated integer or group of integers but not the exclusion of any other integer or group of integers.

Other definitions for selected terms used herein may be found within the detailed description of the invention and apply throughout. Unless otherwise defined, all other scientific and technical terms used herein have the same meaning as commonly understood to one of ordinary skill in the art to which the invention belongs.

The invention described herein may include one or more range of values (for example, size, displacement and field strength etc.). A range of values will be understood to include all values within the range, including the values defining the range, and values adjacent to the range that lead to the same or substantially the same outcome as the values immediately adjacent to that value which defines the boundary to the range. For example, a person skilled in the field will understand that a 10% variation in upper or lower limits of a range can be totally appropriate and is encompassed by the invention. More particularly, the variation in upper or lower limits of a range will be 5% or as is commonly recognised in the art, whichever is greater.

Throughout this specification relative language such as the words 'about' and 'approximately' are used. This language seeks to incorporate 10% variability to the specified number or range. That variability may be plus 10% or negative 10% of the particular number specified

Features of the invention will now be discussed with reference to the following non-limiting description and examples.

Accordingly, the present disclosure provides a method of treating kidney neoplasia in a subject in need of treatment, by subjecting the patient to SIRT.

According an aspect of the disclosure there is provided a method of treatment for renal neoplastic conditions in a subject comprising subjecting the subject to SIRT, wherein (i) the prescribed activity of the irradiated microparticles used in the selective internal radiation therapy is 0.02 to 3.5 GBq and (ii) the therapy delivers between 75 and 800 Gy to the site of treatment in the kidney. Preferably the site of treatment is restricted the neoplasia.

In a second aspect of the disclosure there is provided a method for treating a kidney neoplasia in a subject in need of treatment comprising the step of: administering to the kidney neoplasia an amount of microparticles that delivers a radiation dose between 100 and 600Gy. The microparticles used in the method should be preferably suitable for selective internal radiation therapy.

In a preferred form of the disclosure the microparticles are suitable for SIRT. Ideally the microparticles have a level of radioactivity that is between about 0.02 to 3.5 GBq. Most preferably the radioactivity is capped at a maximum of 3 GBq.

Accordingly, in an embodiment of the disclosure, the radioactivity of the microparticles used in the SIRT is calculated by determining the tumour volume and then adjusting the amount of the radioactive microparticles, having regard to tumour volume, to deliver to the kidney neoplasia a radiation dose of between about 100 and 600Gy.

In a highly preferred form of the disclosure the microparticles provide 3.0GBq (+/-10%). The microparticles are preferably suspended in sterile water for injection. Each vial of 3.0GBq is in a volume of 5ml (microparticles and water together). This allows the required activity of the radionucleotide to be manipulated as a volume.

Preferably the microparticles are irradiated with yttrium-90.

The present disclosure provides a method of treating neoplasia in a subject in need of treatment. As used herein, "neoplasia" refers to the uncontrolled and progressive multiplication of cells under conditions that would not elicit, or would cause cessation of, multiplication of normal cells. Neoplasia results in the formation of a "neoplasm", which is defined herein to mean any new and abnormal growth, particularly a new growth of tissue, in which the growth is uncontrolled and progressive. Malignant neoplasms are distinguished from benign in that the former show a greater degree of anaplasia, or loss of differentiation and orientation of cells, and have the properties of invasion and metastasis. Thus, neoplasia includes "cancer", which herein refers to a proliferation of cells having the unique trait of loss of normal controls, resulting in unregulated growth, lack of differentiation, local tissue invasion, and metastasis. Neoplasias for which the present invention will be particularly useful include, without limitation, renal cell carcinomas.

As used herein "treatment" includes:

(i) preventing a disease, disorder or condition from occurring in an subject which may be predisposed to the disease, disorder and/or condition but has not yet been diagnosed as having it;

(ii) inhibiting the disease, disorder or condition, i.e., arresting its development; or

(iii) relieving the disease, disorder or condition, i.e., causing regression of the disease, disorder and/or condition.

According to the method of the disclosure the subject is preferably a mammal (e.g., human beings, domestic animals, and commercial animals, including cows, dogs, monkeys, mice, pigs, and rats), and is most preferably a human.

**SIRT**

Radiotherapy usually relies on treatment through external beam technologies or more recently through locally administering radioactive materials to patients with cancer as a form of therapy. In some of these, the radioactive materials have been incorporated into small particles, seeds, wires and similar related configurations that can be directly implanted into the cancer. When radioactive particles are administered into the blood supply of the target organ, the technique has become known as Selective Internal Radiation Therapy (**SIRT**). Generally, the main form of application of SIRT has been its use to treat cancers in the liver.

There are many potential advantages of SIRT over conventional, external beam radiotherapy. Firstly, the radiation is delivered preferentially to the cancer within the target organ. Secondly, the radiation is slowly and continually delivered as the radionuclide decays. Thirdly, by manipulating the arterial blood supply with vasoactive substances, it is possible to enhance the percentage of radioactive particles that go to the cancerous part of the organ, as opposed to the healthy normal tissues. This has the effect of preferentially increasing the radiation dose to the cancer while maintaining the radiation dose to the normal tissues at a lower level.

The technique of SIRT has been previously reported (see, for example, Chamberlain M, et al (1983) Brit. J. Surg., 70: 596-598; Burton MA, et al (1989) Europ. J. Cancer Clin. Oncol., 25, 1487-1491; Fox RA, et al (1991) Int. J. Rad. Oncol. Biol. Phys. 21, 463-467; Ho S et al (1996) Europ J Nuclear Med. 23, 947-952; Yorke E, et al (1999) Clinical Cancer Res, 5 (Suppl), 3024-3030; Gray BN, et al. (1990) Int. J. Rad. Oncol. Biol. Phys, 18, 619-623). Treatment with SIRT has been shown to result in high response rates for patients with neoplastic growth in particular with colorectal liver metastases (Gray B.N. et al (1989) Surg. Oncol, 42, 192-196; Gray B, et al. (1992) Aust NZ J Surgery, 62, 105-110; Gray B N et al. (2000) GI Cancer, 3(4), 249-257; Stubbs R, et al (1998) Hepato-gastroenterology Suppl II, LXXVII). Other studies have shown that SIRT therapy can also be effective in causing regression and prolonged survival for patients with primary hepatocellular cancer (Lau W, et al (1994) Brit J Cancer 70, 994-999; Lau W, et al. (1998) Int J Rad Oncol Biol Phys. 40, 583-592). Although SIRT is effective in controlling the liver disease, it is not thought to have an extra-hepatic effect.

SIRT, which may also be known as radio-embolization or microparticle brachytherapy involves two procedural components:

- Embolization: injection into the arterial tumour feeding vessels of permanently embolic microparticles which act as the delivery vehicle for the therapeutic moiety, and
- Irradiation: embolization of microparticles in the distal microvasculature of the tumour delivers high dose irradiation to the tumour microvascular plexus and to tumour cells themselves.

Relevantly, direct irradiation of tissue and microvascular bed destruction, rather than pure embolization is responsible for the tissue destructive effects of SIRT therapy.

Broadly speaking radioactive microparticles do not exhibit pharmacodynamics in the classic sense, but induce cell damage by emitting radiation. Once implanted, radioactive microparticles remain within the vasculature of tumours. They are not phagocytised nor do they dissolve or degrade after implantation. High radiation emitted from the radioactive microparticles is preferably cytocidal to cells within the range of the radiation. After the radioactive microparticle has decayed, the non-radioactive microparticles remain intact and are not removed from the body.

Intrinsic to the concept of SIRT is the preferential placement of the radioactive microparticles selectively into the distal microvascular supply of tumours. This may be achieved by direct injection of the microparticles or through the manipulation of blood flow into and out of the target organ.

Accordingly administration of radionuclide microparticles may be by any suitable means, but preferably by delivery to the relevant artery. For example in treating RCC, administration is preferably by laparotomy to expose the renal artery.

Pre or co-administration of another agent may prepare the tumour for receipt of the particulate material, for example a vasoactive substance, such as angiotension-2 to redirect arterial blood flow into the tumour. Delivery of the particulate matter may be by single or multiple doses, until the desired level of radiation is reached.

**Microparticles**

Preferably the particulate material is substantially spherical, but need not be regular or symmetrical in shape.

The microparticles also need not be limited to any particular form or type of microparticle. Any microparticles may be used in the present invention provided the particles are capable of receiving a radionuclide such as through impregnation, absorbing, coating or more generally bonding the particles together.

The microparticles are prepared as polymeric particles. In another form of the disclosure the microparticles are prepared as ceramic particles (including glass).

Where the microparticles are prepared as a polymeric matrix there are a range of methods that may be used to prepare such particles. By way of example a description of such particles including methods for their production and formulation as well as their use is provided in co-owned European application number 20010978014,

- Where the microparticles are ceramic particles (including glass) the selected particles will usually possess the following properties:
- the particles will generally be biocompatible, such as calcium phosphate-based biomedical ceramics or glass.
- the particles will generally comprise a radionuclide that preferably has sufficiently high energy and an appropriate penetration distance, which are capable of releasing their entire energy complement within the tumour tissue to effectively kill the cancer cells and to minimize damage to adjacent normal cells or to attending medical personnel. The level of radiation activity of the ceramic or glass will be selected and fixed based upon the need for therapy given the particular cancer involved and its level of advancement. The ideal half-life of the radionuclides is somewhere between days and months. On the one hand, it is impractical to treat tumours with radionuclides having too short a half-life, this characteristic limiting therapy efficiency. On the other hand, in radiotherapy it is generally difficult to trace and control radionuclides having a long half-life.
- the particles must be of a suitable size. The size of the particles for treatment depends upon such variables as the surface area of the tumour, capillary permeability, and the selected method of introduction into the tumour (i.v. versus implant by surgical operation).
- some ceramic processes involve inclusion of extraneous substances as contaminants that might produce undesired radionuclides. Should these be well taken care of, the size of the particles can then be controlled by granulation and meshing.

6

There are many processes for producing small granular ceramic or glass particles. One of these involves the introduction of small amounts of the ceramic particles passing through a high-temperature melting region. Ceramic spherules are yielded by surface tension during melting. After the solidification, condensation, collection and sorting processes, ceramic spherules of various sizes can be obtained. The particle size of ceramic spheroid can be controlled by the mass of granules introduced into the high-temperature melting region or can be controlled by collecting spheroids of various sizes through the selection of sedimentary time during liquid-sedimentation.

The ceramic or glass materials for preparing those particles can be obtained commercially or from ultra-pure ceramic raw materials if the commercial products do not meet specifications for one reason or another. The ceramic or glass particles for radiation exposure in this disclosure can be yielded by traditional ceramic processes, which are well known by those skilled in this art. The ceramic processes such as solid-state reaction, chemical co-precipitation, sol-gel, hydrothermal synthesis, glass melting, granulation, and spray pyrolysis can be applied in this disclosure for the production of specific particles.

The ceramic or glass particles of suitable size which are obtained commercially or which are produced by the processes described above are washed twice with distilled water. Then the supernatant is decanted after sedimentation for 3 minutes. The above two steps are repeated 3 times to remove the micro-granules adhering on the surfaces of the particles. Then a certain amount of ceramic or glass particles prepared from the processes described above are introduced into a quartz tube. After being sealed, the quartz tube is placed inside a plastic irradiation tube, then the irradiation tube is closed. The irradiation tube is put into a vertical tube of the nuclear reactor and the multiple tube assembly is irradiated with an approximated neutron flux for an approximated exposed period (e.g., for about 24 to about 30 hours). Following exposure, the irradiation tube is taken out of the nuclear reactor for cooling. According to this method, ceramic or glass particles carrying radionuclides can be generated.

The microparticles of the disclosure, be they polymer or ceramic based, can be separated by filtration or other means known in the art to obtain a population of microparticles of a particular size range that is preferred for a particular use. The size and shape of the microparticles is a factor in the distribution and drug delivery in the tissues.

When microparticles or other small particles are administered into the arterial blood supply of a target organ, it is desirable to have them of a size, shape and density that results in the optimal homogeneous distribution within the target organ. If the microparticles or small particles do not distribute evenly, and as a function of the absolute arterial blood flow, then they may accumulate in excessive numbers in some areas and cause focal areas of excessive radiation.

The ideal particle for injection into the blood stream has a very narrow size range with an SD of less than 5%, so as to assist in even distribution of the microparticles within the target organ, particularly within the kidney and would be sized in the range 5-200 micron, preferably 15-100 micron, and preferably 20-60 micron, and most preferably 30-35 micron.

If the particles are too dense or heavy, then they will not distribute evenly in the target organ and will accumulate in excessive concentrations in areas that do not contain the neoplastic growth. It has been shown that solid, heavy microparticles distribute poorly within the parenchyma of the liver when injected into the arterial supply of the liver. This, in turn, decreases the effective radiation reaching the neoplastic growth in the target organ, which decreases the ability of the radioactive microparticles to kill the tumour cells. In contrast, lighter microparticles with a specific gravity of the order of 2.0 distribute well within the liver. The particulate material is preferably low density, more particularly a density below 3.0 g/cc, even more preferably below 2.8g/cc, 2.5g/cc, 2.3g/cc, 2.2g/cc or 2.0 g/cc.

**Radioactive Particulate Material**

For radioactive particulate material to be used successfully for the treatment of neoplastic growth, the radiation emitted should be of high energy and short range. This ensures that the energy emitted will be deposited into the tissues immediately around the particulate material and not into tissues that are not the target of the radiation treatment. In this treatment mode, it is desirable to have high energy but short penetration beta-radiation, which will confine the radiation effects to the immediate vicinity of the particulate material. There are many radionuclides that can be incorporated into microparticles that can be used for SIRT. Of particular suitability for use in this form of treatment is the unstable isotope of yttrium (Y-90). Yttrium-90 is a high-energy pure beta-emitting isotope with no primary gamma emission. The maximum energy of the beta particles is 2.27MeV, with a mean of 0.93MeV. The maximum range of emissions in tissue is 11mm, with a mean of 2.5mm. The half-life of yttrium-90 is 64.1 hours. In use requiring the isotope to decay to infinity, 94% of the radiation is delivered in 11 days leaving only background radiation with no therapeutic value. The microparticles themselves are a permanent implant and each device is for single patient use.

The radionuclide which is incorporated into the microparticle in accordance with the present invention is yttrium-90, but may also be any other suitable radionuclide which can be precipitated in solution, of which the isotopes of lutetium, holmium, samarium, iodine, phosphorous, iridium and rhenium are some examples.

Preferably the radionuclide is stably incorporated into the particulate material or polymeric matrix such that the incorporated radionuclide does not substantially leach out of the particulate material under physiological conditions such as in the patient or in storage. The leaching of radionuclides from the particular material can cause nonspecific radiation of the patient and damage surrounding tissue. Preferably, the amount of leaching is less than 5%, more preferably less than 4%, 3%, 2%, 1% or 0.9%, 0.8%, 0.7%, 0.6%, 0.5%, 0.4%, 0.3%, 0.2% or 0.1%. One method of assessing leaching is by adjusting a sample to pH 7.0 and agitating in a water bath at 37°C for 20 minutes. A 100$\mu$L sample is counted for beta emission in a Geiger-Müller counter. Another representative 100$\mu$L sample is filtered through a 0.22$\mu$m filter and the filtrate counted for beta emission in the Geiger-Müller counter. The percent unbound radionuclide is calculated by:

$$\frac{FiltrateCount}{SampleCount} \; X \; 100 = \% \, UnboundRadionuclide$$

Desirably, the radionuclide is stably incorporated into the microparticle.

In a preferred form of the invention the microparticle is prepared as a particulate material comprising a polymeric matrix, which is an ion exchange resin, particularly a cation exchange resin. Preferably the ion exchange resin comprises a partially cross linked aliphatic polymer, including polystyrene. One particularly preferred cation exchange resin is the styrene/divinylbenzene copolymer resin commercially available under the trade name Aminex 50W-X4 (Biorad, Hercules, CA). However, there are many other commercially available cation exchange resins that are suitable, including styrene/divinylbenzene copolymer resin with varying degrees of cross-linking.

It is also desirable to have the particulate material manufactured so that the suspending solution has a pH less than 9. If the pH is greater than 9 then this may result in irritation of the blood vessels when the suspension is injected into the artery or target organ. Preferably the pH is less than 8.5 or 8.0 and more preferably less than 7.5.

According to the invention the person skilled in the art will appreciate that SIRT may be applied by any of a range of different methods, some of which are described in US patents 4789501, 5011677, 5302369, 6296831, 6379648, or WO applications 200045826, 200234298 or 200234300.

In one embodiment, the method of the present disclosure is carried out by firstly irradiating yttria (yttrium oxide) in a neutron beam to activate yttria to the isotope yttrium-90. The yttrium-90 oxide is then solubilised, for example as yttrium-90 sulphate solution. The ion exchange resin is preferably provided in the form of an aqueous slurry of microparticles of ion exchange resin having a particle size 30 to 35 microns, and the yttrium-90 sulphate solution is added to the slurry to absorb the yttrium-90 into the ion exchange resin microparticles. Subsequently, the yttrium-90 is precipitated, for example by addition of tri-sodium phosphate solution, to stably incorporate the yttrium-90 into the microparticles. The particulate material may be combined with a solution of the radionuclide or the salt of the radionuclide may be combined with the particulate matter, in a solution suitable for solubilising the radionuclide.

Alternate sources of yttrium-90 may be used in the production of these microparticles. For example, a highly pure source of yttrium-90 may be obtained by extracting yttrium-90 from a parent nuclide and using this extracted yttrium-90 as the source of the soluble yttrium salt that is then incorporated into the polymeric matrix of the microparticles. For example, the method of the present disclosure is carried out by sourcing yttrium-90 from a generator, such as a $^{90}$SR/$^{90}$Y generator

In order to decrease the pH of the suspension containing the microparticles for injection into patients the microparticles may be washed to remove any un-precipitated or loosely adherent radionuclide. According to the method of the present disclosure the microparticles used in the method are prepared as a suspension at the required pH by precipitating the yttrium with a tri-sodium phosphate solution at a concentration containing at least a three-fold excess of phosphate ion, but not exceeding a 30-fold excess of phosphate ion, and then washing the microparticles with deionised water. Another approach, which ensures that the pH of the microparticle suspension is in the desired range, is to wash the resin with a phosphate buffer solution of the desired pH.

## Radioactivity of the Particulate Material

The amount of microparticles used in the method and which will be required to provide effective treatment of a neoplastic growth will depend substantially on the radionuclide used in the preparation of the microparticles.

By way of example, an amount of yttrium-90 activity that will result in an inferred radiation dose to a RCC will be approximately 3.0GBq because the radiation from SIRT is delivered as a series of discrete point sources, the dose

The inventors have revealed that in the treatment of neoplasia, treatment is most effective when the activity of yttrium-90 microparticles is approximately 0.02 to 3.5 GBq and those particles deliver a radiation dose of between about 200 and 800 Gy.

Preferably, the activity of the microparticles is 0.02, 0.03, 0.04, 0.05, 0.06, 0.08, 0.09, 0.10, 0.11, 0.12, 0.14, 0.15, 0.16, 0.17, 0.18, 0.20, 0.21, 0.22, 0.23, 0.24, 0.26, 0.27, 0.28, 0.29, 0.30, 0.32, 0.33, 0.34, 0.35, 0.36, 0.38, 0.39, 0.40, 0.41, 0.42, 0.43, 0.44, 0.45, 0.47, 0.48, 0.49, 0.50, 0.51, 0.52, 0.53, 0.54, 0.55, 0.56, 0.57, 0.59, 0.60, 0.61, 0.63, 0.64, 0.65, 0.66, 0.67, 0.68, 0.69, 0.70, 0.71, 0.72, 0.73, 0.76, 0.78, 0.79, 0.80, 0.81, 0.82, 0.84, 0.85, 0.88, 0.89, 0.90, 0.91, 0.93, 0.94, 0.96, 0.97, 1.00, 1.01, 1.02, 1.03, 1.04, 1.05, 1.06, 1.08, 1.09, 1.1, 1.12, 1.13, 1.14, 1.17, 1.20, 1.2, 1.21, 1.22, 1.25, 1.28, 1.29, 1.3, 1.33, 1.34, 1.36, 1.37, 1.40, 1.4, 1.41, 1.44, 1.45, 1.46, 1.5, 1.52, 1.58, 1.6, 1.62, 1.64, 1.70, 1.7, 1.76, 1.78, 1.8, 1.82, 1.86, 1.88, 1.9, 1.94, 2.00, 2.0, 2.02, 2.06, 2.10, 2.1, 2.12, 2.18, 2.2, 2.26, 2.3, 2.34, 2.4, 2.42, 2.50, 2.5, 2.58, 2.6, 2.66, 2.7, 2.74, 2.8, 2.82, 2.90, 2.9, 3.0, 3.1, 3.2, 3.3, 3.4, 3.5, 3.6, 3.7, 3.8 or 3.9 GBq and the radiation dose delivered to the neoplasia is radiation dose of between about 100 and 800 Gy. More specifically, the activity of the microparticles is 2.6, 2.7, 2.8, 2.9, 3.0, 3.1, 3.2, 3.3 or 3.4 GBq when the radiation dose delivered to the neoplasia is radiation dose of between about 100 and 800 Gy. According to a highly preferred form of the invention the maximum prescribed activity is capped at about 3.0GBq.

In accordance with the invention, SIRT delivers an intended radiation dose to the neoplasia in the kidney of between about 200 and 800 Gy. Preferably, the intended radiation dose delivered to the neoplasia in the kidney is between about 300 and 600 Gy. More preferably, the radiation dose delivered to the neoplasm in the kidney is selected from the following 300, 350, 400, 450, 500, 550, 600 Gy and all radiation doses in between.

Variation to the activity of the microparticle used in the SIRT and the intended radiation dose to the neoplasia are two of the variable that must be accounted for in delivering a therapy. Relevantly, any variation of the radiation dose delivered to the neoplasia will cause a consequential variation to the activity of the microparticles used in the method and vice versa.

In determining the intended radiation dose to a neoplasia in the kidney, a number of other factors must also be taken into account. Those factors include 1) kidney-lung shunting, and 2) the neoplasm volume.

Kidney to lung shunting is the relative amount of irradiated microparticles that pass from the kidney to the lung as a consequence of microparticles failing to lodge in the neoplasm in the kidney. The kidney-to-lung shunt fraction may be determined using any suitable method available in the literature. For example the kidney-to-lung shunt fraction may be determined from a baseline Tc-99m nuclear medicine lung shunt study as described herein. Once the percentage of shunting is determined the volume of microparticles at a prescribed activity required to deliver an intended radiation dose to a kidney neoplasis can be determined by taking account of the percentage loss of particles to shunting.

The kidney neoplasis volume is preferably determined from a baseline MRI based 3-D volume reconstruction scan of the abdomen and pelvis. Such a reconstruction is carried out by MeVis Distant Services, Bremen, Germany. The tumour volume was first determined from the screening (i.e. baseline) MRI.

Preferably the prescribed activity of the irradiated microparticles used in the SIRT of the tumour was calculated is determined by:

    i. identifying the kidney-to-lung shunt fraction of the patient;
    ii. the tumour volume (cc), which is determined from a baseline MRI based 3-D volume reconstruction; and
    iii. the intended radiation dose to tumour (Gy).

The following three tables list the prescribed activity of irradiated microparticles injected into the renal artery or its branches at different lung shunt fractions, different tumour volumes and where the intended radiation dose to tumour is between 75 and 400Gy.

Table 1: Prescribed activity of SIR-Spheres microparticles for patients with a kidney-to-lung shunt fraction of 0% - 10%.

| Tumour Volume (cc) | Intended Radiation Dose to Tumour | | | | | |
|---|---|---|---|---|---|---|
| | Cohort 1: 75Gy | Cohort 2: 100Gy | Cohort 3: 150Gy | Cohort 4: 200Gy | Cohort 5: 300Gy | Cohort 6: 400Gy |
| 10 | 0.02 | 0.02 | 0.03 | 0.04 | 0.06 | 0.08 |
| 20 | 0.03 | 0.04 | 0.06 | 0.08 | 0.12 | 0.16 |
| 30 | 0.05 | 0.06 | 0.09 | 0.12 | 0.18 | 0.24 |
| 40 | 0.06 | 0.08 | 0.12 | 0.16 | 0.24 | 0.32 |
| 50 | 0.08 | 0.10 | 0.15 | 0.20 | 0.30 | 0.40 |
| 60 | 0.09 | 0.12 | 0.18 | 0.24 | 0.36 | 0.48 |
| 70 | 0.11 | 0.14 | 0.21 | 0.28 | 0.42 | 0.56 |
| 80 | 0.12 | 0.16 | 0.24 | 0.32 | 0.48 | 0.64 |
| 90 | 0.14 | 0.18 | 0.27 | 0.36 | 0.54 | 0.72 |
| 100 | 0.15 | 0.20 | 0.30 | 0.40 | 0.60 | 0.80 |

(continued)

| Tumour Volume (cc) | Intended Radiation Dose to Tumour | | | | | |
|---|---|---|---|---|---|---|
| | Cohort 1: 75Gy | Cohort 2: 100Gy | Cohort 3: 150Gy | Cohort 4: 200Gy | Cohort 5: 300Gy | Cohort 6: 400Gy |
| 110 | 0.17 | 0.22 | 0.33 | 0.44 | 0.66 | 0.88 |
| 120 | 0.18 | 0.24 | 0.36 | 0.48 | 0.72 | 0.96 |
| 130 | 0.20 | 0.26 | 0.39 | 0.52 | 0.78 | 1.04 |
| 140 | 0.21 | 0.28 | 0.42 | 0.56 | 0.84 | 1.12 |
| 150 | 0.23 | 0.30 | 0.45 | 0.60 | 0.90 | 1.20 |
| 160 | 0.24 | 0.32 | 0.48 | 0.64 | 0.96 | 1.28 |
| 170 | 0.26 | 0.34 | 0.51 | 0.68 | 1.02 | 1.36 |
| 180 | 0.27 | 0.36 | 0.54 | 0.72 | 1.08 | 1.44 |
| 190 | 0.29 | 0.38 | 0.57 | 0.76 | 1.14 | 1.52 |
| 200 | 0.30 | 0.41 | 0.61 | 0.81 | 1.22 | 1.62 |
| 210 | 0.32 | 0.43 | 0.64 | 0.85 | 1.28 | 1.70 |
| 220 | 0.33 | 0.45 | 0.67 | 0.89 | 1.34 | 1.78 |
| 230 | 0.35 | 0.47 | 0.70 | 0.93 | 1.40 | 1.86 |
| 240 | 0.36 | 0.49 | 0.73 | 0.97 | 1.46 | 1.94 |
| 250 | 0.38 | 0.51 | 0.76 | 1.01 | 1.52 | 2.02 |
| 260 | 0.39 | 0.53 | 0.79 | 1.05 | 1.58 | 2.10 |
| 270 | 0.41 | 0.55 | 0.82 | 1.09 | 1.64 | 2.18 |
| 280 | 0.42 | 0.57 | 0.85 | 1.13 | 1.70 | 2.26 |
| 290 | 0.44 | 0.59 | 0.88 | 1.17 | 1.76 | 2.34 |
| 300 | 0.45 | 0.61 | 0.91 | 1.21 | 1.82 | 2.42 |
| 310 | 0.47 | 0.63 | 0.94 | 1.25 | 1.88 | 2.50 |
| 320 | 0.48 | 0.65 | 0.97 | 1.29 | 1.94 | 2.58 |
| 330 | 0.50 | 0.67 | 1.00 | 1.33 | 2.00 | 2.66 |
| 340 | 0.51 | 0.69 | 1.03 | 1.37 | 2.06 | 2.74 |
| 350 | 0.53 | 0.71 | 1.06 | 1.41 | 2.12 | 2.82 |
| 360 | 0.54 | 0.73 | 1.09 | 1.45 | 2.18 | 2.90 |
| 370 | 0.56 | 0.75 | 1.12 | 1.49 | 2.24 | 2.98 |
| 380 | 0.57 | 0.77 | 1.15 | 1.53 | 2.30 | 3.00 |
| 390 | 0.59 | 0.79 | 1.18 | 1.57 | 2.36 | 3.00 |
| 400 | 0.60 | 0.81 | 1.21 | 1.61 | 2.42 | 3.00 |
| 410 | 0.62 | 0.83 | 1.24 | 1.65 | 2.48 | 3.00 |
| 420 | 0.63 | 0.85 | 1.27 | 1.69 | 2.54 | 3.00 |
| 430 | 0.65 | 0.87 | 1.30 | 1.73 | 2.60 | 3.00 |
| 440 | 0.66 | 0.89 | 1.33 | 1.77 | 2.66 | 3.00 |
| 450 | 0.68 | 0.91 | 1.36 | 1.81 | 2.72 | 3.00 |
| 460 | 0.69 | 0.93 | 1.39 | 1.85 | 2.78 | 3.00 |

(continued)

| Tumour Volume (cc) | Intended Radiation Dose to Tumour | | | | | |
|---|---|---|---|---|---|---|
| | Cohort 1: 75Gy | Cohort 2: 100Gy | Cohort 3: 150Gy | Cohort 4: 200Gy | Cohort 5: 300Gy | Cohort 6: 400Gy |
| 470 | 0.71 | 0.95 | 1.42 | 1.89 | 2.84 | 3.00 |
| 480 | 0.72 | 0.97 | 1.45 | 1.93 | 2.90 | 3.00 |
| 490 | 0.74 | 0.99 | 1.48 | 1.97 | 2.96 | 3.00 |
| 500 | 0.75 | 1.01 | 1.51 | 2.01 | 3.00 | 3.00 |
| 510 | 0.77 | 1.03 | 1.54 | 2.05 | 3.00 | 3.00 |
| 520 | 0.78 | 1.05 | 1.57 | 2.09 | 3.00 | 3.00 |
| 530 | 0.80 | 1.07 | 1.60 | 2.13 | 3.00 | 3.00 |
| 540 | 0.81 | 1.09 | 1.63 | 2.17 | 3.00 | 3.00 |
| 550 | 0.83 | 1.11 | 1.66 | 2.21 | 3.00 | 3.00 |
| 560 | 0.84 | 1.13 | 1.69 | 2.25 | 3.00 | 3.00 |
| 570 | 0.86 | 1.15 | 1.72 | 2.29 | 3.00 | 3.00 |
| 580 | 0.87 | 1.17 | 1.75 | 2.33 | 3.00 | 3.00 |
| 590 | 0.89 | 1.19 | 1.78 | 2.37 | 3.00 | 3.00 |
| 600 | 0.91 | 1.21 | 1.82 | 2.42 | 3.00 | 3.00 |

Table 2: Prescribed activity of SIR-Spheres microparticles for patients with a kidney-to-lung shunt fraction of 11% - 15%.

| Tumour Volume (cc) | Intended Radiation Dose to Tumour | | | | | |
|---|---|---|---|---|---|---|
| | Cohort 1: 75Gy | Cohort 2: 100Gy | Cohort 3: 150Gy | Cohort 4: 200Gy | Cohort 5: 300Gy | Cohort 6: 400Gy |
| 10 | 0.02 | 0.02 | 0.03 | 0.04 | 0.06 | 0.08 |
| 20 | 0.03 | 0.04 | 0.06 | 0.08 | 0.12 | 0.16 |
| 30 | 0.05 | 0.06 | 0.09 | 0.12 | 0.18 | 0.24 |
| 40 | 0.06 | 0.08 | 0.12 | 0.16 | 0.24 | 0.32 |
| 50 | 0.08 | 0.10 | 0.15 | 0.20 | 0.30 | 0.40 |
| 60 | 0.09 | 0.12 | 0.18 | 0.24 | 0.36 | 0.48 |
| 70 | 0.11 | 0.14 | 0.21 | 0.28 | 0.42 | 0.56 |
| 80 | 0.12 | 0.16 | 0.24 | 0.32 | 0.48 | 0.64 |
| 90 | 0.14 | 0.18 | 0.27 | 0.36 | 0.54 | 0.72 |
| 100 | 0.15 | 0.20 | 0.30 | 0.40 | 0.60 | 0.80 |
| 110 | 0.17 | 0.22 | 0.33 | 0.44 | 0.66 | 0.88 |
| 120 | 0.18 | 0.24 | 0.36 | 0.48 | 0.72 | 0.96 |
| 130 | 0.20 | 0.26 | 0.39 | 0.52 | 0.78 | 1.04 |
| 140 | 0.21 | 0.28 | 0.42 | 0.56 | 0.84 | 1.12 |
| 150 | 0.23 | 0.30 | 0.45 | 0.60 | 0.90 | 1.20 |
| 160 | 0.24 | 0.32 | 0.48 | 0.64 | 0.96 | 1.28 |

(continued)

| Tumour Volume (cc) | Intended Radiation Dose to Tumour | | | | | |
|---|---|---|---|---|---|---|
| | Cohort 1: 75Gy | Cohort 2: 100Gy | Cohort 3: 150Gy | Cohort 4: 200Gy | Cohort 5: 300Gy | Cohort 6: 400Gy |
| 170 | 0.26 | 0.34 | 0.51 | 0.68 | 1.02 | 1.36 |
| 180 | 0.27 | 0.36 | 0.54 | 0.72 | 1.08 | 1.44 |
| 190 | 0.29 | 0.38 | 0.57 | 0.76 | 1.14 | 1.52 |
| 200 | 0.30 | 0.41 | 0.61 | 0.81 | 1.22 | 1.62 |
| 210 | 0.32 | 0.43 | 0.64 | 0.85 | 1.28 | 1.70 |
| 220 | 0.33 | 0.45 | 0.67 | 0.89 | 1.34 | 1.78 |
| 230 | 0.35 | 0.47 | 0.70 | 0.93 | 1.40 | 1.86 |
| 240 | 0.36 | 0.49 | 0.73 | 0.97 | 1.46 | 1.94 |
| 250 | 0.38 | 0.51 | 0.76 | 1.01 | 1.52 | 2.02 |
| 260 | 0.39 | 0.53 | 0.79 | 1.05 | 1.58 | 2.10 |
| 270 | 0.41 | 0.55 | 0.82 | 1.09 | 1.64 | 2.18 |
| 280 | 0.42 | 0.57 | 0.85 | 1.13 | 1.70 | 2.26 |
| 290 | 0.44 | 0.59 | 0.88 | 1.17 | 1.76 | 2.34 |
| 300 | 0.45 | 0.61 | 0.91 | 1.21 | 1.82 | 2.42 |
| 310 | 0.47 | 0.63 | 0.94 | 1.25 | 1.88 | 2.50 |
| 320 | 0.48 | 0.65 | 0.97 | 1.29 | 1.94 | 2.58 |
| 330 | 0.50 | 0.67 | 1.00 | 1.33 | 2.00 | 2.66 |
| 340 | 0.51 | 0.69 | 1.03 | 1.37 | 2.06 | 2.74 |
| 350 | 0.53 | 0.71 | 1.06 | 1.41 | 2.12 | 2.74 |
| 360 | 0.54 | 0.73 | 1.09 | 1.45 | 2.18 | 2.74 |
| 370 | 0.56 | 0.75 | 1.12 | 1.49 | 2.24 | 2.74 |
| 380 | 0.57 | 0.77 | 1.15 | 1.53 | 2.30 | 2.74 |
| 390 | 0.59 | 0.79 | 1.18 | 1.57 | 2.36 | 2.74 |
| 400 | 0.60 | 0.81 | 1.21 | 1.61 | 2.42 | 2.74 |
| 410 | 0.62 | 0.83 | 1.24 | 1.65 | 2.48 | 2.74 |
| 420 | 0.63 | 0.85 | 1.27 | 1.69 | 2.54 | 2.74 |
| 430 | 0.65 | 0.87 | 1.30 | 1.73 | 2.60 | 2.74 |
| 440 | 0.66 | 0.89 | 1.33 | 1.77 | 2.66 | 2.74 |
| 450 | 0.68 | 0.91 | 1.36 | 1.81 | 2.72 | 2.74 |
| 460 | 0.69 | 0.93 | 1.39 | 1.85 | 2.72 | 2.74 |
| 470 | 0.71 | 0.95 | 1.42 | 1.89 | 2.72 | 2.74 |
| 480 | 0.72 | 0.97 | 1.45 | 1.93 | 2.72 | 2.74 |
| 490 | 0.74 | 0.99 | 1.48 | 1.97 | 2.72 | 2.74 |
| 500 | 0.75 | 1.01 | 1.51 | 2.02 | 2.72 | 2.74 |
| 510 | 0.77 | 1.03 | 1.54 | 2.06 | 2.72 | 2.74 |
| 520 | 0.78 | 1.05 | 1.57 | 2.10 | 2.72 | 2.74 |

(continued)

| Tumour Volume (cc) | Intended Radiation Dose to Tumour | | | | | |
|---|---|---|---|---|---|---|
| | Cohort 1: 75Gy | Cohort 2: 100Gy | Cohort 3: 150Gy | Cohort 4: 200Gy | Cohort 5: 300Gy | Cohort 6: 400Gy |
| 530 | 0.80 | 1.07 | 1.60 | 2.14 | 2.72 | 2.74 |
| 540 | 0.81 | 1.09 | 1.63 | 2.18 | 2.72 | 2.74 |
| 550 | 0.83 | 1.11 | 1.66 | 2.22 | 2.72 | 2.74 |
| 560 | 0.84 | 1.13 | 1.69 | 2.26 | 2.72 | 2.74 |
| 570 | 0.86 | 1.15 | 1.72 | 2.30 | 2.72 | 2.74 |
| 580 | 0.87 | 1.17 | 1.75 | 2.34 | 2.72 | 2.74 |
| 590 | 0.89 | 1.19 | 1.78 | 2.38 | 2.72 | 2.74 |
| 600 | 0.91 | 1.21 | 1.82 | 2.42 | 2.72 | 2.74 |

Table 3: Prescribed activity of SIR-Spheres microparticles for patients with a kidney-to-lung shunt fraction of 16 - 20%.

| Tumour Volume (cc) | Intended Radiation Dose to Tumour | | | | | |
|---|---|---|---|---|---|---|
| | Cohort 1: 75Gy | Cohort 2: 100Gy | Cohort 3: 150Gy | Cohort 4: 200Gy | Cohort 5: 300Gy | Cohort 6: 400Gy |
| 10 | 0.02 | 0.02 | 0.03 | 0.04 | 0.06 | 0.08 |
| 20 | 0.03 | 0.04 | 0.06 | 0.08 | 0.12 | 0.16 |
| 30 | 0.05 | 0.06 | 0.09 | 0.12 | 0.18 | 0.24 |
| 40 | 0.06 | 0.08 | 0.12 | 0.16 | 0.24 | 0.32 |
| 50 | 0.08 | 0.10 | 0.15 | 0.20 | 0.30 | 0.40 |
| 60 | 0.09 | 0.12 | 0.18 | 0.24 | 0.36 | 0.48 |
| 70 | 0.11 | 0.14 | 0.21 | 0.28 | 0.42 | 0.56 |
| 80 | 0.12 | 0.16 | 0.24 | 0.32 | 0.48 | 0.64 |
| 90 | 0.14 | 0.18 | 0.27 | 0.36 | 0.54 | 0.72 |
| 100 | 0.15 | 0.20 | 0.30 | 0.40 | 0.60 | 0.80 |
| 110 | 0.17 | 0.22 | 0.33 | 0.44 | 0.66 | 0.88 |
| 120 | 0.18 | 0.24 | 0.36 | 0.48 | 0.72 | 0.96 |
| 130 | 0.20 | 0.26 | 0.39 | 0.52 | 0.78 | 1.04 |
| 140 | 0.21 | 0.28 | 0.42 | 0.56 | 0.84 | 1.12 |
| 150 | 0.23 | 0.30 | 0.45 | 0.60 | 0.90 | 1.20 |
| 160 | 0.24 | 0.32 | 0.48 | 0.64 | 0.96 | 1.28 |
| 170 | 0.26 | 0.34 | 0.51 | 0.68 | 1.02 | 1.36 |
| 180 | 0.27 | 0.36 | 0.54 | 0.72 | 1.08 | 1.44 |
| 190 | 0.29 | 0.38 | 0.57 | 0.76 | 1.14 | 1.52 |
| 200 | 0.30 | 0.41 | 0.61 | 0.81 | 1.22 | 1.62 |
| 210 | 0.32 | 0.43 | 0.64 | 0.85 | 1.28 | 1.70 |
| 220 | 0.33 | 0.45 | 0.67 | 0.89 | 1.34 | 1.78 |
| 230 | 0.35 | 0.47 | 0.70 | 0.93 | 1.40 | 1.86 |

(continued)

| Tumour Volume (cc) | Intended Radiation Dose to Tumour | | | | | |
|---|---|---|---|---|---|---|
| | Cohort 1: 75Gy | Cohort 2: 100Gy | Cohort 3: 150Gy | Cohort 4: 200Gy | Cohort 5: 300Gy | Cohort 6: 400Gy |
| 240 | 0.36 | 0.49 | 0.73 | 0.97 | 1.46 | 1.94 |
| 250 | 0.38 | 0.51 | 0.76 | 1.01 | 1.52 | 2.02 |
| 260 | 0.39 | 0.53 | 0.79 | 1.05 | 1.58 | 2.06 |
| 270 | 0.41 | 0.55 | 0.82 | 1.09 | 1.64 | 2.06 |
| 280 | 0.42 | 0.57 | 0.85 | 1.13 | 1.70 | 2.06 |
| 290 | 0.44 | 0.59 | 0.88 | 1.17 | 1.76 | 2.06 |
| 300 | 0.45 | 0.61 | 0.91 | 1.21 | 1.82 | 2.06 |
| 310 | 0.47 | 0.63 | 0.94 | 1.25 | 1.88 | 2.06 |
| 320 | 0.48 | 0.65 | 0.97 | 1.29 | 1.94 | 2.06 |
| 330 | 0.50 | 0.67 | 1.00 | 1.33 | 2.00 | 2.06 |
| 340 | 0.51 | 0.69 | 1.03 | 1.37 | 2.06 | 2.06 |
| 350 | 0.53 | 0.71 | 1.06 | 1.41 | 2.06 | 2.06 |
| 360 | 0.54 | 0.73 | 1.09 | 1.45 | 2.06 | 2.06 |
| 370 | 0.56 | 0.75 | 1.12 | 1.49 | 2.06 | 2.06 |
| 380 | 0.57 | 0.77 | 1.15 | 1.53 | 2.06 | 2.06 |
| 390 | 0.59 | 0.79 | 1.18 | 1.57 | 2.06 | 2.06 |
| 400 | 0.60 | 0.81 | 1.21 | 1.61 | 2.06 | 2.06 |
| 410 | 0.62 | 0.83 | 1.24 | 1.65 | 2.06 | 2.06 |
| 420 | 0.63 | 0.85 | 1.27 | 1.690 | 2.06 | 2.06 |
| 430 | 0.65 | 0.87 | 1.30 | 1.73 | 2.06 | 2.06 |
| 440 | 0.66 | 0.89 | 1.33 | 1.77 | 2.06 | 2.06 |
| 450 | 0.68 | 0.91 | 1.36 | 1.81 | 2.06 | 2.06 |
| 460 | 0.69 | 0.93 | 1.39 | 1.85 | 2.06 | 2.06 |
| 470 | 0.71 | 0.95 | 1.42 | 1.89 | 2.06 | 2.06 |
| 480 | 0.72 | 0.97 | 1.45 | 1.93 | 2.06 | 2.06 |
| 490 | 0.74 | 0.99 | 1.48 | 1.97 | 2.06 | 2.06 |
| 500 | 0.75 | 1.01 | 1.50 | 2.02 | 2.06 | 2.06 |
| 510 | 0.77 | 1.03 | 1.50 | 2.06 | 2.06 | 2.06 |
| 520 | 0.78 | 1.05 | 1.50 | 2.06 | 2.06 | 2.06 |
| 530 | 0.80 | 1.07 | 1.50 | 2.06 | 2.06 | 2.06 |
| 540 | 0.81 | 1.09 | 1.50 | 2.06 | 2.06 | 2.06 |
| 550 | 0.83 | 1.11 | 1.50 | 2.06 | 2.06 | 2.06 |
| 560 | 0.84 | 1.13 | 1.50 | 2.06 | 2.06 | 2.06 |
| 570 | 0.86 | 1.15 | 1.50 | 2.06 | 2.06 | 2.06 |
| 580 | 0.87 | 1.17 | 1.50 | 2.06 | 2.06 | 2.06 |
| 590 | 0.89 | 1.19 | 1.50 | 2.06 | 2.06 | 2.06 |

(continued)

| Tumour Volume (cc) | Intended Radiation Dose to Tumour | | | | | |
|---|---|---|---|---|---|---|
| | Cohort 1: 75Gy | Cohort 2: 100Gy | Cohort 3: 150Gy | Cohort 4: 200Gy | Cohort 5: 300Gy | Cohort 6: 400Gy |
| 600 | 0.91 | 1.21 | 1.50 | 2.06 | 2.06 | 2.06 |

According to the method of the disclosure, each delivery consists of sufficient microparticles to provide 3.0GBq (+/-10%) on the day of calibration. The microparticles are preferably suspended in sterile water or like physiological solution for injection. Each vial of 3.0GBq is dispatched in a volume of 5ml (microparticles and water together). This allows the required activity of the radionucleotide to be manipulated as a volume.

**Other Cytotoxic Agents**

Desirably microparticles have the potential to interact with other cytotoxic agents and are typically administered concomitantly with either systemic or loco-regional chemotherapeutic agents such as oxiplatin, 5-Fluorouricil or Leucovorin. This interaction may be exploited to the benefit of the patient, in that there can be an additive toxicity on tumour cells, which can enhance the tumour cell kill rate. This interaction can also lead to additive toxicity on non-tumourous cells.

In addition to the identified chemotherapeutic agents and radionuclide microparticles the disclosure may also include an effect treatment of immunomodulators as part of the therapy. Illustrative immunomodulators suitable for use in the invention are alpha interferon, beta interferon, gamma interferon, interleukin-2, interleukin-3, tumour necrosis factor, granulocyte-macrophage colony stimulating factors, and the like.

The present disclosure further provides a synergistic combination of antineoplastic agents and an amount of radionuclide-doped microparticles suitable for use in SIRT for treatment of a neoplastic growth. Preferably, the combination is prepared for use in treating a patient with RCC metastases.

The disclosure also relates to pharmaceutical composition comprising an effective antineoplastic agent and an amount of radionuclide microparticles suitable for use in SIRT for treatment of a neoplastic growth. Preferably, the pharmaceutical composition is prepared for use in treating a patient with RCC metastases.

The disclosure further relates to a kit for killing RCC in a subject. The kit comprises an effective amount of an antineoplastic agent and an amount of radionuclide microparticles as described above suitable for use in SIRT for treatment of RCC growth. The kit may further comprise an instructional material.

Further features of the present disclosure are more fully described in the following Examples. It is to be understood, however, that this detailed description is included solely for the purposes of exemplifying the present invention, and should not be understood in any way as a restriction on the broad description of the invention as set out above.

**EXAMPLES**

Initially a pre-clinical study of selective internal radiation therapy - also known as radioembolisation - of the porcine kidney was undertaken to examine the technical feasibility and in vivo safety of delivering SIR-Spheres microparticles within a large animal renal application.

The purpose of this animal study was to determine whether super-selective delivery of SIR-Spheres microparticles to the porcine kidney was technically feasible, and to evaluate the histopathological changes in the treatment target zone (upper or lower renal pole), the adjacent non-targeted renal tissue, and adjacent and distant organs following administration of SIR-Spheres microparticles, compared with bland resin microparticles which served as a control.

Super-selective delivery of SIR-Spheres microparticles was made to one kidney and an equivalent number of bland microparticles were administered to the corresponding pole of the contralateral kidney as a control. The aim of treatment was to implant a prescribed (i.e. predetermined) amount of yttrium-90 activity to a target zone equivalent to approximately one-third of the kidney volume. The macroscopic and microscopic changes to the kidney and to adjacent and distant tissues resulting from incremental increases in implanted activity (between 0.15GBq and 0.35GBq) in each of 6 animals were graded in a blinded manner by a pathologist with specialist training in renal pathology.

In this study grade 4 histological changes were recorded in the treatment target zone (upper or lower renal pole) in 5 of 6 animals following super-selective injection of SIR-Spheres microparticles, with evidence of nephron-sparing effects in the adjacent renal tissue at the lowest activities. With activities beyond 0.3GBq, increasing damage was observed in the adjacent renal tissue beyond the treatment target zone due to the intentionally complete embolization of the microvasculature in the treatment target zone, stasis of antegrade flow in the renal artery, followed by retrograde flow (i.e. reflux) with "spillover" of microparticles into adjacent renal tissue. Importantly, renal function as measured by serum creatinine remained within the normal range in all animals, even at the highest implanted activities. Furthermore, there were no toxicities evident in adjacent or distant organs, or systemically and no acute or delayed adverse reactions occurred in any of the animals

**Pilot study in humans to treat RCC**

Initially a pilot study was conducted in humans to evaluate the feasibility, safety, toxicity and potential effectiveness of selective internal radiation therapy (SIRT) using SIR-Spheres microparticles as a treatment for patients with renal cell carcinoma that is not suitable for curative therapy by conventional means. Patients were recruited serially into six dose escalating cohorts: 75Gy, 100Gy, 150Gy, 200Gy, 300Gy, 400Gy intended radiation dose to tumour.

This pilot study evaluated the use of selective internal radiation therapy (SIRT) using SIR-Spheres microparticles as a treatment for patients with renal cell carcinoma that is not suitable for curative therapy by conventional means.

**Biocompatibility Safety Data**

The following summarises the biocompatibility data on file at Sirtex and with regulatory authorities as follows:

- FDA in PMA 990065 Vols. 5 & 6,
- EU held by BSI in Design Dossier relating to certificate CE 70318.
- TGA in File DV-2006-3529.

As a medical device the biocompatibility profile of SIR-Spheres microparticles is relatively benign. The following tests were carried out to ensure the safety of the device. These tests, where relevant were performed on both labelled and non-radioactive microparticles. Non-irradiated SIR-Spheres microparticles were assessed both in animals and in vitro for the following:

- Haemocompatibility (in vitro) (tested to ISO 10993-4)
- Mammalian cell cytogenicity (in vitro) (Chinese hamster ovary cells)
- Cytotoxicity (in vitro) (tested to ISO 10993-5)
- Bacterial reverse mutation test (in vitro) (OECD 471 & 472)
- Maximum sensitisation (guinea pig) (tested to ISO 10993-10)
- Intracutaneous toxicity (reactivity) (rabbit) (tested to ISO 10993-10)
- Systemic toxicity of potential leach products (mouse) 9tested to ISO 10993-11).

In summary, SIR-Spheres microparticles are haemocompatible, non-cytotoxic, non-mutagenic, are non-toxic locally or systemically and are a mild sensitiser in the guinea pig under the conditions of the test. The details for the tests are presented in greater detail below. All tests were carried out on microparticles labelled with inert yttrium. Radioactive microparticles are cytocidal, hence any potential toxicity of the polymer or yttrium itself are masked.

**Toxicology**

All testing was conducted in compliance with GLP in compliance with the OECD Principles of GLP (ISBN 9264-12367-9-1982).

**Localised Toxicity**

Localised toxicity was assessed with the intracutaneous injection test in the rabbit (ISO 10993-10, March 1995). This test was conducted with a 50% v/v dilution of the microparticles in water, as the standard presentation will not traverse an intradermal needle. Three female New Zealand white rabbits were used and each rabbit had 5 x 0.2ml of the test device injected intradermally on one side of the midline of the back and 5 x 0.2ml of water for injection as the controls on the other side. At the completion of the observation period (72 hours) the primary irritation scores and the primary irritation index were calculated as per ISO 10993-10. There was negligible response to the device indicating that it is not locally irritant or toxic.

**Systemic Toxicity**

Systemic toxicity was assessed with the systemic injection test in the mouse. The methodology was from ISO 10993-11 biological evaluation of medical devices part 11: tests for systemic toxicity and also the United States Pharmacopoeia 23 1995 for assessment of biological reactivity, *in-vivo*, section 88, page 1699. This test was conducted to evaluate systemic responses to extracts of the microparticles following intravenous and intraperitoneal injection.

Polar (water for injection) and non-polar (cottonseed oil) extracts were prepared. Blanks of both extracts were also prepared. A fifth solution (solution A), being neat supernatant from centrifuged microparticles was also used. The four extract preparations were each tested in five mice, all of which received only a single systemic injection. Solution A was tested in four mice. All doses were 50 ml/kg. Animals were observed over a 72 hour period for signs of toxicity. There were no differences between blanks and extracts and all animals in all groups maintained weight and a healthy appearance throughout. Intravenous administration of the water for injection in which the microparticles are supplied failed to produce apparent toxic effects. Under the conditions of this study, SIR-Spheres microparticles do not leach or produce any toxic substances that are released systemically.

**Mutagenicity**

Mutagenicity was assessed using the bacterial reverse mutation test utilising the strains *Salmonella typhimurium* TA 1535, TA1537, TA 98 and TA 100, and *Escherichia coli* WP2 uvrA. This test assesses the mutagenicity of a substance by its ability to revert specified bacterial strains from auxotrophic growth to prototrophy. It was conducted according to the requirements of the OECD regulatory guideline for testing chemicals, OECD 471 and 472 adopted May 26th 1983.

Positive controls consisted of direct acting mutagens and those that require metabolic activation. Direct mutagens were sodium azide, 9-aminoacrindine, 2-nitrofluorene and cumene hydroperoxide for *S. typhimurium* and 4-nitroquinoline-N-oxide for *E. coli.* The metabolically activated mutagen was 2-aminoanthracene for both bacterial strains. Rat cytochrome P450 mitochondrial fraction was the metabolic activation system used. The methodology involved initially using a plate. If this was positive, the second experiment was also with a plate, but if negative, then preincubation was used. The mean and the standard deviation of the plate counts for each experiment were calculated and statistically assessed using a Dunnett's test. A positive result was a statistically significant increase in the numbers of revertants scored in two separate experiments. A negative result was no greater increases in revertants than may be expected from normal variation for any strain in either experiment.

All positive controls gave results in the expected ranges indicating the strains used were sensitive to mutagens. There were no statistically significant increases in revertants from SIR-Spheres microparticles, thus this device was not mutagenic under the conditions of the test.

Mutagenicity was also assessed using an *in vitro* cytogenetic test, which determines if mutagenicity (if present) is due to structural chromosomal damage. This was performed in mammalian cells (Chinese hamster ovary cells). Mutagenicity after metabolic activation of the test substance was also assessed by using the rat cytochrome P450 mitochondrial fraction. Positive controls were mitomycin C (direct mutagen) and benzo(a)pyrene and cyclophosphamide were the metabolically activated mutagens. Scoring of chromosomal damage was by the ISCN classification. Any increase in number of aberrations was compared to negative control using a Fisher's Exact test. The positive controls caused statistically significant increases in aberrations scored, indicating sensitivity of the test system. Under the conditions of this test SIR-Spheres microparticles were not clastogenic.

**Cytotoxicity**

Cytotoxicity was assessed by an *in vitro cytotoxicity test*, which assessed the potential cytotoxicity of leachable endogenous or extraneous substances on the microparticles. The cell lines used were mouse fibroblast L929 (ATCC, CCL1, NCTC clone 929). Phenol was the positive control and neat minimum essential medium (MEM) was the negative. Cells were examined microscopically after incubation with dilutions of the supernatant (water for injection) from the microparticles. The dilutions of supernatant used were 0.5%-2% v/v.

Under the conditions of this test, the microparticles leached no substance that altered cell morphology or caused any cytotoxic effects at concentrations of 0.5, 1.0 and 5.0mg/ml.

**Haemocompatibility**

Haemocompatibility was assessed according to ISO 10993-4 *'Selection of Tests for Interactions with Blood'.* The positive control was de-ionised water and the negative was normal saline. These results were in the expected ranges. The cell line was human erythrocytes. Solutions of whole blood with supernatant from the microparticles, as well as solutions of microparticles from 0.5mg/mL to 5.0mg/mL were tested. After incubation, the test tubes were centrifuged and assessed spectrophotometrically at 545 nm. Under the conditions of this test, less than 5% haemolysis was considered non-haemolytic. Neither the supernatant from the microparticles or solutions of microparticles were haemolytic. A 5.0mg/ml solution of microparticles is approximately iso-osmolar with normal saline.

Under the conditions of this test, any potentially leachable substances in or on the microparticles had no haemolytic activity against human erythrocytes.

### Sensitising Ability

Sensitising ability was assessed with the *maximum sensitisation test* in the guinea pig. This test evaluated the potential of the device to cause a delayed dermal hypersensitivity / type 1V immune response. This test was conducted on methodology of ISO 10993-10 Biological Evaluation of Medical Devices: Test for Irritation and Sensitisation of March 1995. The test was conducted on 20 test female albino guinea pigs and 10 controls. The topical range finding study in four animals indicated that the microparticles were non-irritant. The lack of primary irritancy allowed assessment for delayed sensitivity. Of those tested, three of the 20 animals gave a positive skin response (grade 1) at 24 or 48 hours after challenge. No animals in the test or control group exhibited a positive reaction to water. The weak positive responses in the test group indicate a delayed dermal hypersensitivity according to criteria in ISO 10993. The device is therefore considered a mild sensitiser under the condition of this test.

### Summary of Preclinical Testing

These test results are evidence of the inert nature of the microparticles *per se*. The therapeutic activity of the device is due to the emission of beta radiation. Neither the polymer nor the yttrium itself contributed to the cell death expected from implantation of the microparticles. The device, once decayed, caused no toxicity when left *in situ* within treated tumours. The implications of the mild sensitising ability of the device to humans are difficult to determine. Clinical experience to date - in excess of 10,000 SIR-Spheres microparticles devices having been implanted into humans for the treatment of liver tumours, as at September 2009 - has not demonstrated a sensitivity reaction to SIR-Spheres microparticles.

### CLINICAL RISK ANALYSIS

### Complications of SIR-Spheres Microparticles

This study represented the first in-human application of SIR-Spheres microparticles for the treatment of primary renal cell carcinoma, no human data were available on the complications from treatment with SIR-Spheres microparticles in this specific disease setting.

However, as SIR-Spheres microparticles are a marketed active implantable device approved for the treatment of inoperable liver tumours, extensive human data are available on the complications of SIR-Spheres microparticles when used for the treatment of hepatic malignancy, which will be described for completeness below.

Over 10,000 treatments have been performed globally with SIR-Spheres microparticles for the management of liver cancer. Overall, the incidence of complications after SIR-Spheres microparticles therapy in broader clinical use, if patients are selected appropriately and target (i.e. liver) delivery is performed meticulously, is low.

Gastrointestinal complications occur in less than 10% of those treated and are largely preventable. Gastric and duodenal ulceration have been reported after SIRT and are related to the inadvertent intestinal deposition of microparticles via extra-hepatic visceral arterial branches. Even in the absence of extra-hepatic activity on Tc-99m labelled MAA and Bremsstrahlung emission images, gastrointestinal symptoms have been reported to develop. The risk of gastrointestinal ulceration can be minimized via the routine coil embolization of the extra-hepatic visceral arteries (e.g. gastro-duodenal, right gastric, supraduodenal arteries) before infusion of SIR-Spheres microparticles.

The gallbladder also may receive SIR-Spheres microparticles through a patient cystic artery, leading to radiation cholecystitis. In order to avoid this potential complication infusion distal to the cystic artery may be possible. However, even with infusion of SIR-Spheres microparticles proximal to the cystic artery, the risk of radiation cholecystitis requiring cholecystectomy is low. This issue is addressed at the time of administration by the treating Interventional Radiologist, via catheter placement and/or selective embolization/optimization of the hepatic arterial vasculature.

A life-threatening complication, progressive pulmonary insufficiency secondary to radiation-induced lung fibrosis can be avoided by excluding from treatment with SIRT any patient with significant liver-to-lung shunting. There have been no reported occurrences of radiation induced lung disease since routine pre-treatment lung shunt quantification using Tc-99m labelled MAA has been standard practice.

Radiation induced liver disease (**RILD**) is a rare complication of SIRT treatment. It results in various degrees of hepatic decompensation and is clinically indistinguishable from hepatic veno-occlusive disease. RILD is manifested clinically by the development of anicteric ascites. High doses of corticosteroids typically are administered in an attempt to decrease intra-hepatic inflammation. Treatment results are variable and mostly of minimal benefit, as the condition will progress in some patients to hepatic insufficiency of various degrees.

Pancytopaenia as a result of bone marrow suppression from leaching of yttrium-90 was reported after the use of the earliest microparticle device (Mantravadi, 1982). The yttrium-90 microparticle device has subsequently undergone multiple revisions and this complication has not been reported since that time. SIR-Spheres microparticles are classified as a sealed-source device.

From the total experience with SIR-Spheres microparticles, major complications have included:

- In approximately one-third of patients, administration of SIRT caused immediate short-term abdominal pain requiring narcotic analgesia and was typically self-limiting.
- Post-SIRT lethargy and nausea were common symptoms and could last up to two weeks and sometimes require medication.
- Most patients developed a mild-moderate fever that lasted for several days following SIRT administration. This fever did not usually require treatment.
- The most common potential serious complications resulted from either:
- inadvertent administration of SIR-Spheres microparticles into the gastrointestinal tract resulting in gastritis/duodenitis, or
- radiation induced liver disease resulting from a radiation overdose to the normal liver parenchyma.

The incidence of gastritis/duodenitis was be reduced by meticulous attention to the administration procedure so as to ensure that there was a minimal chance of SIR-Spheres microparticles entering the numerous small arteries supplying the gastrointestinal tract. Radiation induced liver disease was largely, but not totally, preventable by using appropriate SIRT doses and making allowances for dose reduction when there was increased risk of causing radiation damage such as in pre-existing liver damage, poor liver reserve or small volume tumour mass in the liver. The reported incidence of gastritis/duodenitis was <10%, while the reported rate of radiation induced liver disease was < 2%.

Rare complications that were reported include acute pancreatitis resulting from SIR-Spheres microparticles refluxing in the hepatic artery and lodging in the pancreas, and liver abscess from infection of necrotic tumour.

Previously reported radiation pneumonitis was not observed where appropriate pre-treatment workup and dose reductions was followed.

The rate of treatment related complications was shown to run at 2 - 10%, with outcomes related to the skill and experience of the Interventional Radiologist and Nuclear Medicine Physician.

**Known Contra-indications to SIR-Spheres Microparticles**

It is established that SIR-Spheres microparticles are contra-indicated (Sirtex Training Manual) in patients who have:

- Had previous external beam radiation therapy to the liver.
- Ascites or other clinical signs of liver failure.
- Abnormal synthetic and excretory liver function tests as determined by serum albumin (must be > 3.0 g/dL) and total bilirubin (must < 2.0 mg/dL), respectively.
- Complete main portal vein thrombosis without cavernous transformation.
- Disseminated extra-hepatic disease.
- Tumours amenable to surgical resection or ablation with intent to cure.
- Greater than 20% lung shunting (as determined by pre-treatment Tc-99m labelled MAA nuclear medicine lung shunt study).
- Pre-assessment angiogram and MAA nuclear medicine scan demonstrating significant and uncorrectable activity in the stomach, pancreas or bowel.
- Been treated with Capecitabine within the previous 8 weeks, or who will be treated with Capecitabine within 8 weeks of treatment with SIR-Spheres microparticles.

**Identifying the Specific Risks of SIR-Spheres Microparticles for the Treatment of Renal Cell Cancer**

While the aforementioned complications from, and contra-indications to SIRT therapy pertain specifically to the treatment of liver cancer, the principal risks that may arise from the use of SIRT within the kidney may relate to:

- Non-targeted delivery of SIR-Spheres microparticles to visceral organs outside of the kidney resulting in unintended radiation damage to such organs, and
- Excessive renal-to-lung shunting resulting in radiation pneumonitis.

In order to mitigate the possible risk of unintended (i.e. non-targeted) delivery of SIR-Spheres microparticles to organs or tissues outside the kidney, only the Interventional Radiologist who performed the normal porcine kidney study (seven animals; Chief Investigator Dr. Simon Mackie; UNSW Animal Care and Ethics Committee (ACEC) Number 09/24B; dated 02/03/2009) delivered renal SIRT therapy in this study protocol. This Interventional Radiologist (Dr Suresh DeSilva) has performed in excess of 50 patient treatments using SIR-Spheres microparticles for liver cancer and is thus highly experienced with this loco-regional therapy. Extensive interventional radiology expertise is required in order to 1) perform meticulously the visceral and renal angiograms and reliably identify any aberrant renal vessels which may be present and which may supply non-renal tissues or organs, and 2) possess the necessary technical expertise to prevent microparticle delivery to these aberrant vessels, *viz.* embolization or distal microparticle injection. Sirtex provided any necessary proctoring as required.

In order to prevent excessive renal-to-lung shunting, and as an additional investigation to identify the presence of non-targeted arterial flow to extra-renal tissues or organs, each patient entered onto the study underwent a renal-to-lung shunt quantification prior to the delivery of SIR-Spheres microparticles. Renal-to-lung shunting was detected using gamma emission scintigraphy with the injection of 180 - 220MBq of Technecium-99m labelled macro-aggregated albumin (Tc-99m-MAA) into the intended renal arterial territory. The renal-to-lung shunt fraction was then calculated as the ratio of the gamma emission count in the lung to that in the kidney in regions of interest in planar scintigrams. The ratio was calculated as a percentage that was rounded to the nearest whole percentage point. Patients in whom the renal-to-lung shunt fraction indicated potential exposure to the lung to an absorbed radiation dose of more than 25Gy were excluded from treatment with SIRT.

Note (1): This strata included patients who had stable disease while on other treatments, e.g. tyrosine kinase inhibitors etc. and patients who declined treatment by conventional techniques.

**Patient Eligibility**

Patients had histologically confirmed renal cell carcinoma of the kidney of any subtype. Patients were stratified as being either not amenable to treatment by conventional techniques, or patients not requiring (who had declined) immediate treatment by conventional techniques, at the time of study entry. Histological specimens were obtained via ultrasound-guided core biopsy of the affected kidney, where feasible.

In order to be considered eligible for the study, patients had to fulfil the inclusion and exclusion criteria specified below.

**Inclusion Criteria**

Patients had to be:
• Willing, able and mentally competent to provide written informed consent.
• Histologically, radiologically or clinically confirmed primary renal cell carcinoma of the kidney.
• Unequivocal and measurable MRI evidence of primary renal cell carcinoma that either:
• was not suitable for treatment by surgical resection, local ablation or other conventional techniques with curative intent;
• did not require (or where the patient had declined) immediate treatment by surgical resection, local ablation or other conventional techniques with curative intent, at the time of study entry. Note: this included those patients who had stable disease while on other treatments, e.g. tyrosine kinase inhibitors etc.

• Note: measurable disease was defined as primary RCC lesions that could be accurately measured in at least one dimension with longest diameter >10 mm using MRI .

• Metastatic disease other than untreated CNS metastases was permitted.
• All imaging evidence used as part of the **screening** process was less than 45 days old at the time of delivery of protocol SIRT therapy.
• Suitable for protocol therapy as determined by both the Medical Oncology and Surgical Urology Investigators.
• Other than radiotherapy, prior therapy for primary renal cell carcinoma was permitted, provided that such therapy was administered and completed at least 45 days prior to entry into the study.
• WHO performance status 0 - 2.
• Adequate haematological and renal function as follows:

| **Haematological** | Neutrophils | $> 1.5 \times 10^9$/L |
| | Platelets | $> 100 \times 10^9$/L |
| **Renal** | Estimated GFR | $> 40$ml/min/1.73m$^2$ or |

(continued)

$\geq$ 35ml/min/1.73m$^2$
provided estimated GFR increased
to $\geq$ 40ml/min/1,73m$^2$ after hydration

Note: It was a requirement that blood results were less than 45 days old at the time of study entry.
Estimated GFR was calculated using the Cockroft-Gault formula and corrected to a body surface area of 1.73m$^2$.

- Aged 18 years or older.
- Female patients were required to be postmenopausal, surgically sterile, or if of child bearing age and sexually active, using an acceptable method of contraception.
- Male patients were required to be surgically sterile or if sexually active and having a pre-menopausal female partner must be using an acceptable method of contraception.
- Life expectancy of at least 3 months without any active treatment.
- Renal arterial anatomy suitable for implantation of SIR-Spheres microparticles, as assessed by visceral and renal angiogram.

**Exclusion Criteria**

Patients were excluded in the following circumstances.

- Previous external beam radiotherapy delivered to the kidney or within a 5cm margin. Note: Patients who had been previously treated with SIR-Spheres microparticles were still eligible for retreatment provided they received SIR-Spheres microparticles more than 6 months previously and did not develop DLT, and benefited from treatment with SIR-Spheres microparticles previously (i.e. at least stable disease over 6 months or better).
- Subsequent therapy was planned to be administered within 32 days of the delivery of protocol SIRT therapy.
- Renal-to-lung shunt fraction that indicated potential exposure to the lung to an absorbed radiation dose of more than 20Gy.
- Inadequate renal function as defined by estimated GFR < 40ml/min/1,7m$^2$ or estimated GFR $\geq$ 35ml/min/1.73m$^2$ that did not increase to $\geq$ 40ml/min/1.73m$^2$ after hydration.
- Intercurrent disease that would render the patient unsuitable for treatment according to the protocol.
- Equivocal, immeasurable, or unevaluable primary renal cell carcinoma in the kidney.
- Pregnant or breast feeding.

**Patient Screening**

All patients referred for possible participation in the study were screened by both the Medical Oncology and Surgical Urology Investigators to confirm the patient's eligibility to receive protocol treatment.

The screening period, during which the patient's eligibility to receive protocol treatment as part of this study was confirmed and defined as the time period between study entry (i.e. signing of the informed consent document) and the commencement of protocol treatment, did not exceed 45 days. Patients only commenced protocol treatment after all eligibility criteria had been confirmed. Where a patient withdrew after study entry but before commencement of protocol treatment for any reason, the patient was re-screened at a later date provided they continued to meet all study eligibility criteria.

**Clinical Assessment**

All patients were assessed clinically by both the Medical Oncology and Surgical Urology Investigators to determine the patient's eligibility to receive protocol treatment. Clinical assessment included a comprehensive medical history and physical examination including weight was required to be completed within 45 days of study entry.

**Haematological and Biochemical Investigations**

All patients were required to undergo the following haematological and biochemical investigations in order to determine their eligibility to receive protocol treatment. These investigations were completed within 45 days of study entry:

| Haematological | Full blood examination (FBE) |
| --- | --- |

(continued)

| | Erythrocyte sedimentation rate (ESR) |
| | C-reactive protein (CRP) |
| Renal | Urea, electrolytes, serum creatinine[1] (UEC) Calcium, magnesium, phosphate, uric acid |
| Liver | Liver function tests (LFTs) |
| Pregnancy test | Serum or urine pregnancy test in female patients |
| Urinalysis | Protein, creatinine |

Note: (1) serum creatinine was recorded (together with patient weight) and the estimated GFR was calculated using the Cockcroft-Gault formula.

### Radiological and Nuclear Medicine Investigations

All patients were required to undergo the following investigations in order to determine their eligibility to receive protocol treatment, and to demonstrate the extent of disease.

### Non-contrast CT Scan of the Chest

A non-contrast spiral CT scan of the chest was used to determine the presence and extent of metastases. The CT series was completed within 45 days of study entry.

### MRI Study of the Abdomen and Pelvis

An MRI study of the abdomen and pelvis was used to determine the extent of kidney disease and to determine the presence and extent of metastases. This MRI series was completed within 45 days of study entry.

### Ultrasound Study of the Kidney

An ultrasound study of the kidney was used to determine the extent of kidney disease and determine the presence and extent of metastases. This ultrasound series was completed within 45 days of study entry.

### DTPA Clearance Study of Glomerular Filtration Rate

Glomerular filtration rate (GFR) was measure by a DTPA clearance study to quantify baseline renal function. The DTPA clearance study was corrected to a body surface area of $1.73m^2$. This DTPA clearance study was completed within 45 days of study entry.

### Assessment of Patient Suitability for Selective Internal Radiation Therapy

All patients were assessed in order to determine their eligibility to receive protocol SIRT therapy. This assessment was completed within 45 days of study entry.

### Visceral and Renal Angiogram

All patients underwent an outpatient diagnostic visceral and renal angiogram to determine the vascular anatomy of the kidney and to perform a nuclear medicine kidney-to-lung shunt study.

The renal angiogram provided a road map of the arterial supply of the kidney and tumour in order to plan the optimal delivery of the SIR-Spheres microparticles. The renal angiogram was performed together with the lung shunt study and the results of these two assessments were assessed prior to implanting the SIR-Spheres microparticles.

The diagnostic renal angiogram was performed in order to:

- Fully identify and define all relevant renal arterial vasculature:
- Aortogram
- Right or left renal artery
- Replaced, accessory and aberrant arteries.
- Confirm the ability to selectively catheterise the renal arterial vasculature.
- Assess the flow characteristics in the renal arteries.

- Determine the renal arterial supply to the tumour(s) i.e. upper pole branch(es), lower pole branch(es), accessory renal arteries, other aberrant arteries.
- Confirm the absence of blood shunting from the kidney to the adrenal gland, the ureters or other abdominal organs that could not be corrected via catheter techniques (coil embolization, placement of temporary balloon occlusion device, etc.). If the renal angiogram indicated an uncorrectable risk of flow to any unintended organs, then protocol SIRT treatment was not administered.
- Perform a technetium-99m macro-aggregated albumin (Tc-99m MAA) lung shunt study to assess the presence and degree of lung shunting from the kidney.

**Tc-99m MAA Lung Shunt Study**

It was expected that in some patients with primary renal cell carcinoma there would be sufficient arterio-venous shunts present in the kidney to allow SIR-Spheres microparticles injected into the kidney to pass through the kidney and lodge in the lungs. As excessive shunting to the lungs might have resulted in radiation damage to the lungs, a nuclear medicine 'break-through' scan was performed in all patients to quantify the extent of kidney-to-lung shunting.

A lung shunt study was conducted to assess arterial perfusion of the kidney and the fraction of radiopharmaceutical tracer that will pass through the kidney and lodge in the lungs. According to the study a dose of 150MBq of Technetium-99m labelled macro-aggregated albumin (MAA) was administered to a patient according the following procedure.

A temporary trans-femoral catheter is placed in the renal artery of a patient at the location intended for implantation of microspheres. The Tc-99m labelled MAA is injected through the catheter into the renal artery. The patient is positioned supine under the gamma camera and the images are recorded. Anterior and posterior images of abdomen and thorax are taken. Approximately 700 - 1000 counts were measured for the abdomen and equivalent time for the thorax.

Using that data the G mean was calculated for kidney region and lung region. From that data the lung/kidney ratio was calculated and then used to determine the applicable table (table 1: 0 - 10%, table 2: 11 - 15%, table 3: 16 - 20%) in Appendix 2 to determine the prescribed activity of SIR-Spheres microspheres.

The percentage of Tc-99m MAA that escaped through the kidney and lodged in the lungs was expressed as a 'percentage lung shunt'. Normally this should be less than 10%. The total lung radiation dose delivered by SIR-Spheres microparticles was required to be ≤25Gy in order to ensure that the patient did not develop radiation induced lung disease.

Table 4 shows the approximate lung radiation dose delivered for different combinations of 1) implanted activity of SIR-Spheres microparticles and 2) percentage kidney-to-lung shunting. The table assumes that the mass of both lungs plus blood is 1000g.

Table 4 Lung radiation dose calculation (Gy delivered to the lungs)

| Implanted activity of SIR-Spheres microparticles (GBq) | Percentage kidney-to-lung shunting | | |
|---|---|---|---|
| | 10 % | 15 % | 20 % |
| 1.0 | 5 | 7.5 | 10 |
| 1.5 | 7.5 | 11.25 | 15 |
| 2.0 | 10 | 15 | 20 |
| 2.5 | 12.5 | 18.75 | 25 |
| 3.0 | 15 | 22.5 | 30 |

**Commencement of Protocol Treatment**

Once patients were screened and deemed eligible to participate in the study, protocol treatment commenced.

**TREATMENT**

Patients began study treatment as soon as possible, but no later than 45 days after study entry. All patients were followed for a period of 12 months or until death.

**Protocol Treatment: SIR-Spheres Microparticles**

**Calculation of Prescribed Activity of SIR-Spheres Microparticles**

Patients were serially recruited into six dose escalating cohorts:

- **Cohort 1:** 75Gy intended radiation dose to tumour
- **Cohort 2:** 100Gy intended radiation dose to tumour
- **Cohort 3:** 150Gy intended radiation dose to tumour
- **Cohort 4:** 200Gy intended radiation dose to tumour
- **Cohort 5:** 300Gy intended radiation dose to tumour
- **Cohort 6:** 400Gy intended radiation dose to tumour

The prescribed activity of SIR-Spheres microparticles to deliver into the feeding renal arterial circulation of the tumour was calculated using the tables below and was determined by:

- the kidney-to-lung shunt fraction of the patient (0-10%, 11-15%, 16-20%) which was determined from the baseline Tc-99m MAA lung shunt study
- the tumour volume (cc) which was determined from the baseline MRI based 3-D volume reconstruction which was performed by MeVis Distant Services, Bremen, Germany
- the intended radiation dose to tumour (Gy) which was determined according to the cohort that the patient was recruited to.

Note that the maximum prescribed activity was capped at 3.0GBq.

The tumour volume was first determined from the screening (i.e. baseline) MRI scan of the abdomen and pelvis. The tumour volume was <u>determined independently</u> by MeVis Distant Services, Bremen, Germany on a 1 working day turnaround basis. DICOM data files were uploaded to MeVis Labs via secure web-link by the Radiologist Investigator. MeVis Labs then provided an Adobe pdf file of the tumour volume and normal kidney volume to the Radiologist Investigator. This document was stored in the patient file and was used as the basis for calculating the patient's prescribed activity of SIR-Spheres microparticles to implant into the renal tumour feeding vessel(s).

Tables 1 to 3 above list the prescribed activity of SIR-Spheres microparticles injected into the renal artery or its branches.

The kidney-to-lung shunt fraction was determined from the baseline Tc-99m nuclear medicine lung shunt study. The tumour volume was determined from the baseline MRI based 3-D volume reconstruction which was performed by MeVis Distant Services, Bremen, Germany. The intended radiation dose to tumour was determined according to the cohort that the patient was recruited to. Note that the maximum prescribed activity was capped at 3.0GBq.

**Administration of SIR-Spheres Microparticles**

SIR-Spheres microparticles were implanted via a temporary trans-femoral renal arterial micro-catheter. The details of the SIR-Spheres microparticles <u>prescribed</u> and <u>actual</u> implanted activity was recorded in the CRF.

The pre-determined end-points for the administration of SIR-Spheres microparticles into the renal arterial circulation were either:

- Administration of the entire prescribed activity of SIR-Spheres microparticles (as calculated from the tables in Appendix 2), or
- Administration of SIR-Spheres microparticles to the point of sluggish antegrade renal arterial flow, at which point further infusion of SIR-Spheres microparticles resulted in completed embolic occlusion of the tumour micro-vascular bed. This point is referred to as "imminent stasis". The stopping point for the infusion of SIR-Spheres microparticles was at the discretion of the treating Interventional Radiologist.

The technique for delivery of SIR-Spheres microparticles was provided in the Sirtex Medical Training Manual.

**Post-SIRT SPECT Study**

Following the administration of SIR-Spheres microparticles, a same day post-SIRT single photon emission computer tomography (**SPECT**) study of the abdomen/pelvis was performed. The SPECT study detects the Bremsstrahlung radiation from the yttrium-90 and was performed in order to confirm the placement of SIR-Spheres microparticles in the kidney and to exclude non-targeted delivery of SIR-Spheres microparticles to extra-renal locations.

**Measurement of Residual Activity Post-Treatment**

Once the pre-determined end-point for the administration of SIR-Spheres microparticles into the renal arterial circulation was reached, the micro-catheter was removed from the patient and the amount of activity remaining in the SIR-Spheres microparticles v-vial, delivery tubing and micro-catheter was assayed, in order to determine the amount of activity that was actually administered to the patient.

This was done by subtracting the residual activity remaining in the delivery equipment from the original prescribed activity, to arrive at an "actual implanted activity". The method for measuring the residual activity of SIR-Spheres microparticles was at the discretion of the Nuclear Medicine Investigator.

The residual activity was measured by using either of two methods: 1) by using equidistant measurements with a G-M probe taken at four positions around the v-vial at 0°, 90°, 180°, 270° prior to, and immediately after treatment; and 2) by placing the v-vial, delivery tubing and micro-catheter back into the dose calibrator (typically a "Capintec 15R") which was used to assay the prescribed activity of SIR-Spheres microparticles during dose preparation. Either method was acceptable in this study protocol.

**Supportive Treatment**

Supportive treatment was administered when required according to the patient's condition. Such supportive treatment included, but was not limited to, anti-emetics, analgesia, corticosteroids, antibiotics etc. All supportive treatment was recorded on the CRF, including any supportive treatment provided for the implantation of SIR-Spheres microparticles.

**Concomitant Medications**

All medications taken by the patient including medications that were unrelated to their cancer management was recorded in the CRF. These include long-term as well as short-term or acute medications ongoing at the time of signature of the informed consent form or started any time after signature of the informed consent form, until 90 days after SIRT was administered.

Routine medications were listed in the appropriate section and were only recorded on the CRF once, unless they were changed. Additional routine medications were recorded on the CRF upon commencement of the new medication. Commencement and cessation dates, dosage and route of administration were recorded.

**Non-Protocol Treatment**

Once protocol treatment (i.e. SIRT) was delivered, the patient received the best available care as determined by the treating Investigator. Patients were permitted to receive further systemic chemotherapy or biologic therapy commencing no earlier than 3 months post-SIRT, at the discretion of the treating Investigator. Details of such therapy was recorded on the follow-up form of the CRF.

**SERIAL STUDY ASESSMENTS**

**Baseline Assessments**

Baseline assessments were described in detail above. Many of the screening investigations required to confirm a patient's eligibility to receive protocol treatment on this study were performed routinely as standard care for patients with renal cell carcinoma. The results were acceptable for baseline assessment if they were taken within the 45 days screening period. The baseline assessments included:

- Medical history and physical examination including weight
- Haematological and biochemical investigations
- Full blood examination (FBE)
- Erythrocyte sedimentation rate (ESR)
- C-reactive protein (CRP)
- Urea, electrolytes, creatinine (UEC)
- Calcium, magnesium, phosphate, uric acid
- Liver functions tests (LFTs)
- Serum or urine pregnancy test in female patients
- Urinalysis
- Radiological and nuclear medicine investigations
- Non-contrast CT scan of the chest
- MRI study of the abdomen and pelvis
- Ultrasound study of the kidney
- DTPA clearance study of GFR renal function

Consenting patients underwent diagnostic visceral and renal angiogram to determine the arterial blood supply to the kidney and tumour and a Tc-99m MAA scan in order to assess the kidney-to-lung shunting.

**Follow-up Assessments**

All patients received their follow-up assessments according to the following study calendar. Additional non-study assessments were performed as clinically indicated at the discretion of the treating Investigator.

All patients were followed for a period of 12 months. In the event of a patient developing disease progression (either in the kidney or at other sites, or both) the patient remained on-study and continued to undergo follow-up until 12 months.

Table 5 Study Calendar

| | Baseline Assessments | Follow-up Assessments: First 30 Days Post-SIRT | | | Follow-up Assessments: 1 - 12 months Post-SIRT |
|---|---|---|---|---|---|
| Schedule | ≤ 45 days prior to protocol SIRT treatment | Day 0: SIRT | Day 14 | Day 30 | Month 3, 6, 9, 12 |
| Informed consent | ✓ | | | | |
| Demographics | ✓ | | | | |
| Medical history, incl. concurrent illnesses - concurrent meds. | ✓ | | | | ✓ |
| Physical exam, incl. weight - performance status | ✓ | ✓ | ✓ | ✓ | ✓ |
| Haematology, biochemistry & urinalysis | ✓ | ✓ | ✓ | ✓ | ✓ |
| Pregnancy test for females | ✓[a] | | | | |
| Non-contrast CT chest | ✓ | | | | ✓ |
| MRI abdomen, pelvis | ✓ | | | ✓ | ✓ |
| Ultrasound kidney | ✓ | | | ✓ | ✓ |
| DTPA clearance study | ✓ | | | | ✓[b] |
| Visceral & renal angiogram[c] | ✓ | | | | |
| Tc-99m MAA lung shunt study[c] | ✓ | | | | |
| SIRT[d] | | ✓ | | | |
| Post-SIRT SPECT study (same day) | | ✓ | | | |
| Adverse events | | ✓ | ✓ | ✓ | ✓ |
| Quality of life[e] | ✓ | | | ✓ | ✓ |
| Survival | | | | | ✓ |

The acceptable tolerances in the time points listed in table 14.2 were:

- Day 0, 14 assessments could be +/- 2 days
- Day 30 assessment could be +/- 5 days
- Every 3 month assessments could be +/- 2 weeks

**RESPONSE ASSESSMENT**

The following criteria was used to assess response to treatment and for the evaluation of study end points.

**Safety and Toxicity (Primary Objective)**

Safety and toxicity was assessed using the NCI Common Terminology Criteria (NCI-CTC) version 4.0 (see Appendix 4). Patients were followed for safety and toxicity from the time of providing informed consent until day 30 post-SIRT. Definitions and requirements in dealing with adverse events (AE) and serious adverse events (SAE).

**Tumour Response (Secondary Objective)**

Responses were calculated using response evaluation criteria in solid tumours (RECIST) criteria.

**RECIST Guidelines**

All measurable lesions (defined as lesions that could be accurately measured in at least one dimension with longest diameter >10 mm using MRI or CT scan) up to a maximum of five lesions per organ with a maximum of 10 lesions in total, representative of all involved organs, were identified as target lesions and were recorded and measured at baseline.
A sum of the longest diameter for all target lesions was calculated and reported as a baseline sum longest diameter (LD). The baseline sum LD was used as the reference with which to characterize the objective tumour response.
All other lesions (or sites of disease) were identified as non-target lesions and were recorded at baseline. Measurements of these lesions was required, but the presence or absence of each was noted throughout follow-up.

**Response Criteria**

Complete Response (CR): Disappearance of all target lesions associated with the disappearance of all non-target lesions. CR was confirmed if determined by two observations not less than 4 weeks apart.
Partial Response (PR): At least a 30% decrease in the sum of the longest diameter of the target lesions, taking as a reference the baseline sum longest diameter, or a CR associated with persistence of non-target lesions.
Progressive Disease (PD): At least 20% increase in the sum of the longest diameter of the target lesions, taking as a reference the smallest sum of the longest diameter recorded since treatment started or the appearance of new lesions.
Stable Disease (SD): Neither sufficient shrinkage to qualify for a partial response nor sufficient increase to qualify for progressive disease, taking as a reference the smallest sum longest diameter since the start of treatment.

**Progression-Free Survival (Secondary Objective)**

Progression-free survival (PFS) was defined as the time interval between study entry and the date of tumour progression. Tumour progression in the kidney was determined from serial MRI scans. Tumour progression at other sites was measured by any definitive imaging technique including CT scan, MRI scan, or ultrasound scan.
The documented date of recurrence was the date of confirmation of the recurrence. At the time of recurrence, investigators were required to clearly indicate the site of tumour recurrence (renal or extra-renal).

**Overall Survival (Secondary Objective)**

All patients were followed-up for a period of 12 months post-SIRT. Overall survival (OS) was defined as the time interval between the date of study entry and the date of death.

**STATISTICAL CONSIDERATIONS & METHODOLOGY**

**Study Design and Sample Size**

This study was the first in human study to evaluate the feasibility, safety, toxicity and potential effectiveness of SIRT as a treatment for patients with renal cell carcinoma that was not suitable for curative therapy by conventional means.
This study was conducted as a radiation dose escalation trial recruited patients in sequential radiation dose escalating cohorts of 3 - 6 patients, depending on the toxicities observed in each cohort. A minimum of 15 patients was required once it was established that there was no undue toxicity within the first three dose levels.

**Radiation Dose Escalation Plan**

The following table 6 describes how the radiation dose to tumour was escalated in successive patient cohorts:

| Cohort Number | Intended Radiation Dose to Tumour | Number of Patients in Cohort |
|---|---|---|
| 1 | 75Gy | 3 - 6 |
| 2 | 100Gy | 3 - 6 |
| 3 | 150Gy | 3 - 6 |
| 4 | 200Gy | 3 - 6 |
| 5 | 300Gy | 3 - 6 |
| 6 | 400Gy | 3 - 6 |

As in traditional dose-escalation study designs, 3 patients were entered at a given radiation dose level. Once it was established that there was no dose limiting toxicity (DLT) evident at a given radiation dose level, then the next 3 patients were entered at the next highest radiation dose level. Radiation doses continued to be escalated for each cohort until DLT was reached.

**Dose Limiting Toxicity**

This was defined as any $\geq$ grade 3 toxicity occurring during the first 30 days after the administration of SIR-Spheres microparticles that were judged as possibly, probably, or certainly related to SIRT therapy, excluding the following adverse events that were commonly associated with SIRT therapy and thus did not constitute due cause for this study to be stopped:

- Abdominal pain
- Nausea
- Vomiting
- Fever

**Maximum Tolerated Dose / Recommended Phase II Dose Level**

The MTD was defined as the highest radiation dose level at which < 1/3 or < 2/6 patients experience DLT within the first 30 days of SIRT therapy. The recommended "Phase II dose level" (RPTD) was either the MTD or, if dose-escalation reached cohort 4, this was the RPTD without formally defining the MTD.

**Claims**

1.  An amount of irradiated microparticles for use in a method of treating renal neoplastic conditions in a subject, said method comprising subjecting the subject to selective internal radiation therapy (SIRT), wherein a prescribed activity of the irradiated microparticles used in the SIRT is 0.02 to 3.5 GBq, and wherein the irradiated microparticles are yttrium-90 polymeric microspheres, **characterised in that** the SIRT delivers a radiation dose between 200 and 800 Gy to a site of treatment in the kidney.

2.  The amount of microparticles for use of claim 1, wherein the site of treatment in the kidney is a kidney neoplasia.

3.  The amount of microparticles for the use of claim 1 or claim 2, wherein the prescribed activity is capped at a maximum of 3 GBq.

4.  The amount of microparticles for the use of claim 2 or claim 3, wherein the prescribed activity of the irradiated microparticles used in the SIRT is calculated by determining the tumour volume and then adjusting the amount of the irradiated microparticles, so as to deliver to the kidney neoplasia a radiation dose between 200 and 800 Gy.

**Patentansprüche**

**1.** Eine Menge an bestrahlten Mikropartikeln zur Verwendung in einem Verfahren zur Behandlung von neoplastischen Nierenerkrankungen in einem Patienten, das besagte Verfahren umfasst den Patienten einer selektiven internen Strahlentherapie (SIRT) zu unterziehen, wobei eine vorgeschriebene Aktivität der bestrahlten Mikropartikel die in der SIRT verwendet werden 0,02 bis 3,5 GBq beträgt, und wobei die bestrahlten Mikropartikel Yttrium-90-Polymer-Mikrokugeln sind, dadurch charakterisiert, dass die SIRT eine Strahlungsdosis zwischen 200 und 800 Gy an einer zu behandelnden Stelle in der Niere abliefern.

**2.** Die Menge an Mikropartikeln zur Verwendung nach Anspruch 1, wobei die Behandlungsstelle in der Niere ein Nierenkrebs ist.

**3.** Die Menge an Mikropartikeln zur Verwendung nach Anspruch 1 oder Anspruch 2, wobei die vorgeschriebene Aktivität bei maximal 3 GBq gekappt ist.

**4.** Die Menge an Mikropartikeln für die Verwendung nach Anspruch 2 oder 3, wobei die vorgeschriebene Aktivität der bestrahlten Mikropartikel die in der SIRT verwendet werden, durch Bestimmung des Tumorvolumens berechnet und dann die Menge der bestrahlten Mikropartikel so eingestellt wird, sodass die Niere mit einer Strahlungsdosis zwischen 200 und 800 Gy bestrahlt wird.

**Revendications**

**1.** Quantité de microparticules irradiées pour utilisation dans un procédé de traitement des troubles rénaux néoplasiques chez un sujet, ledit procédé consistant à soumettre le sujet à une thérapie de radiation interne sélective (SIRT), une activité prescrite des microparticules irradiées utilisées dans la SIRT étant de 0,02 à 3,5 GBq, et les microparticules irradiées étant des microsphères polymères de l'yttrium-90, **caractérisé en ce que** la SIRT fournie une dose de radiation entre 200 et 800 Gy à un site de traitement dans le rein.

**2.** Quantité de microparticules pour utilisation selon la revendication 1, le site de traitement dans le rein étant une néoplasie rénale.

**3.** Quantité de microparticules pour utilisation selon la revendication 1 ou 2, l'activité prescrite étant plafonnée à un maximum de 3 GBq.

**4.** Quantité de microparticules pour utilisation selon la revendication 2 ou 3, l'activité prescrite des microparticules irradiées utilisées dans la SIRT étant calculée en déterminant le volume de la tumeur et ensuite ajustant la quantité des microparticules irradiées pour fournir à la néoplasie rénale une dose de radiation entre 200 et 800 Gy.

## REFERENCES CITED IN THE DESCRIPTION

**Patent documents cited in the description**

- EP 20010978014 A
- US 4789501 A
- US 5011677 A
- US 5302369 A
- US 6296831 B
- US 6379648 B
- WO 200045826 A
- US 200234298 B
- US 200234300 B

**Non-patent literature cited in the description**

- **MACKIE S.** Superselective radioembolization of the porcine kidney with Yttrium-90 resin microspheres: A feasibility, safety and dose-ranging study. *CARDIOVASCULAR AND INTERVENTIONAL RADIOLOGY,* 2010, vol. 33 (2), 285-290
- **CHAMBERLAIN M et al.** *Brit. J. Surg.,* 1983, vol. 70, 596-598
- **BURTON MA et al.** *Europ. J. Cancer Clin. Oncol.,* 1989, vol. 25, 1487-1491
- **FOX RA et al.** *Int. J. Rad. Oncol. Biol. Phys.,* 1991, vol. 21, 463-467
- **HO S et al.** *Europ J Nuclear Med.,* 1996, vol. 23, 947-952
- **YORKE E et al.** *Clinical Cancer Res,* 1999, vol. 5, 3024-3030
- **GRAY BN et al.** *Int. J. Rad. Oncol. Biol. Phys,* 1990, vol. 18, 619-623
- **GRAY B.N. et al.** *Surg. Oncol,* 1989, vol. 42, 192-196
- **GRAY B et al.** *Aust NZ J Surgery,* 1992, vol. 62, 105-110
- **GRAY B N et al.** *GI Cancer,* 2000, vol. 3 (4), 249-257
- **STUBBS R et al.** *Hepato-gastroenterology,* 1998
- **LAU W et al.** *Brit J Cancer,* 1994, vol. 70, 994-999
- **LAU W et al.** *Int J Rad Oncol Biol Phys.,* 1998, vol. 40, 583-592
- *United States Pharmacopoeia,* 1995, vol. 23